# EUROPEAN PATENT APPLICATION

(11) **EP 4 506 350 A1**
(43) Date of publication of application: **12.02.2025**
(21) Application number: 23190307.1
(22) Date of filing: 08.08.2023
(51) Int. Cl.: C07F 11/00, C07C 17/26, C07C 37/48, C07C 201/12, C07C 209/68, C07C 247/08, C07C 253/30, C07D 213/127, B01J 32/00, B01J 21/00, C07C 22/04, C07C 39/21, C07C 205/06, C07C 211/54, C07C 255/51, C07C 211/23

(54) **AIR-STABLE MOLYBDENUM ALKYLIDYNE CATALYST, PROCESS FOR THE PREPARATION THEREOF, AND THE USE THEREOF FOR HIGH PERFORMANCE ALKYNE METATHESIS**

(71) Applicant: Studiengesellschaft Kohle gGmbH, 45470 Mülheim (DE)
(72) Inventor: FÜRSTNER, Alois, 45478 Mülheim (DE); KORBER, Johannes Nepomuk, 45479 Mülheim (DE); WILLE, Christian, 45149 Essen (DE)

(57) **Abstract**

The present invention refers to new air-stable molybdenum alkylidyne catalysts, a process for the preparation thereof and their use for high performance alkyne metathesis.

## Description

The present invention refers to new air-stable molybdenum alkylidyne catalysts, a process for the preparation thereof, and their use for high performance alkyne metathesis.

During the last two decades, alkyne metathesis has arguably evolved from a curiosity to a method of strategy level status in material science and small molecule synthesis alike (J. Am. Chem. Soc. 2021, 143 (38), 15538-15555). The progress is innately linked to advances in catalyst design. Most catalysts used today are molybdenum alkylidynes endowed with adequate ancillary ligands such as fluorinated alkoxides, amides or silanolates; however, none of these catalysts are bench-stable. Among them, molybdenum alkylidynes endowed with a tripodal silanolate ligand sphere belong to the most active and selective catalysts for alkyne metathesis known to date (J. Am. Chem. Soc. 2020, 142, 11279-11294); they are not bench-stable either.

Notwithstanding the high level of sophistication in the field, a greater user-friendliness therefore remains a desideratum yet to be fully met; only if the best catalysts are easy to handle are they going to be routinely used. Some milestones in this direction have been reached in the past. While molybdenum alkylidynes endowed with silanolate ligands themselves degrade within hours when kept in air, the present laboratory showed that adducts thereof with phenanthroline or 2,2'-bipyridine can be stored at the bench for extended periods of time (Chem. Eur. J. 2012, 18, 10281-10299). However, such adducts show no catalytic activity; prior to use, they must be re-activated by removal of the phenanthroline ligand with the aid of a Lewis-acidic additive such as ZnCl₂ or MnCl₂.

The underlying concept was recently extended by the group of Buchmeiser (Eur. J. Inorg. Chem. 2023, 26, e202200649; Organometallics 2021, 40, 1178-1184) using N-heterocyclic carbenes (NHC's) instead of phenanthroline to form meta-stable adducts. In the solid state, a particular complex of that type was found to have a lifetime of more than a week on the bench; therefore it can be weighed and transferred in air. The alkyne metathesis reaction then needs to be carried out in 1,2-dichloroethane under nitrogen atmosphere at elevated temperature (80°C) to enforce partial decomplexation of the stabilizing NHC ligand, whereas no activity was observed at room temperature. Under these conditions, good turnover numbers were secured at low catalyst loadings, at least for barely functionalized substrates.

Thus, there is still the need for improved alkylidyne catalysts that show high stability in air at the bench and exhibit high catalytic activity without the need for an extra activation step or the use of high temperatures. Surprisingly, the present inventors have found that such properties can be provided by a newly designed tripodal molybdenum alkylidyne tris-silanolate catalyst.

Thus, the present invention is directed to a metal-organic compound of the general Formula (I), 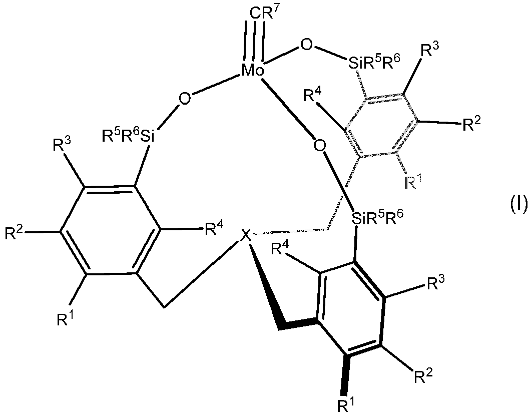
X represents N, CR^{x}, SiR^{x}, wherein R^{x} is selected from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more heteroatoms;
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl, or
(R1 and R2) or (R2 and R3) on the same phenyl ring form an aromatic or heteroaromatic ring structure conjugated with the phenyl ring and having a total of 10 to 18 carbon atoms in the ring structure wherein one to three ring carbon atoms may be replaced by nitrogen, which aromatic or heteroaromatic ring structure may be substituted by one or more heteroatoms,
R4 is selected from H, methyl or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, which alkyl, cycloalkyl, aromatic or heteroaromatic ring structure may be substituted by one or more substituents selected from C1 to C6 alkyl, 6-14 membered aryl or heteroatoms,
R7 is selected from C1-C12-alkyl, C3-C12-cycloalkyl, 6-18 membered aryl, or 5-10 membered heteroaryl which alkyl, cycloalkyl, aromatic or heteroaromatic ring structure may be substituted by one or more substituents selected from C1 to C6 alkyl, 6-14 membered aryl or heteroatoms.

In another embodiment, the present invention refers to a metal-organic compound of Formula (I): 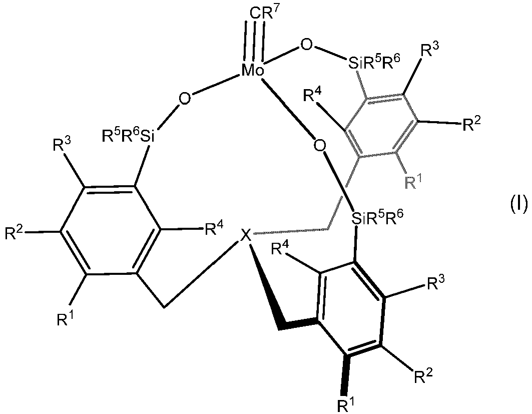 Wherein:
X represents N, CR^{x}, SiR^{x}, wherein R^{x} is selected from C1-C12-alkyl, C3-C12-cycloalkyl, 6-18 membered aryl, or 5-10 membered heteroaryl,
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl,
R4 is selected from H, methyl, or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, 6-18 membered aryl, or 5-10 membered heteroaryl, which alkyl, cycloalkyl, aromatic or heteroaromatic ring structure may be substituted by one or more substituents selected from C1 to C6 alkyl, 6-14 membered aryl or heteroatoms
R7 is selected from C1-C12-alkyl, C3-C12-cycloalkyl or 6-18 membered aryl which alkyl, cycloalkyl, or aromatic ring structure may be substituted by one or more substituents selected from C1 to C6 alkyl, 6-14 membered aryl or heteroatoms.

In a still further embodiment, the invention refers to a metal-organic compound of Formula (I): 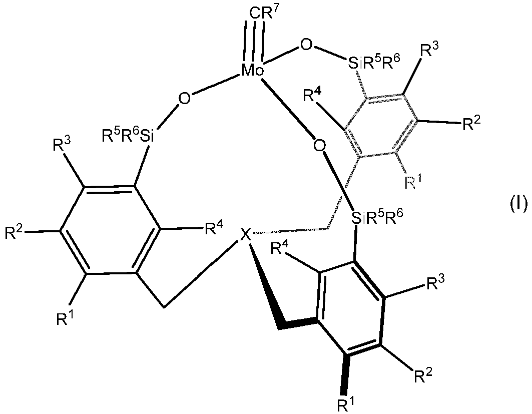 Wherein
X is N,
R1, R2 and R3 may be equal or different from one another and are selected independently from H, C1-C12-alkyl, or C3-C12-cycloalkyl,
R4 is selected from H, methyl or ethyl,
R5 and R6 may be equal or different from one another and are selected independently from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more substituents selected from C1 to C6 alkyl, 6-14 membered aryl or heteroatoms
R7 is selected from C1-C12-alkyl or 6-18 membered aryl, which alkyl or aromatic ring structure may be substituted by one or more substituents selected from C1 to C6 alkyl, 6-14 membered aryl or heteroatoms.

In yet another embodiment, the invention refers to a metal-organic compound of Formula (I): 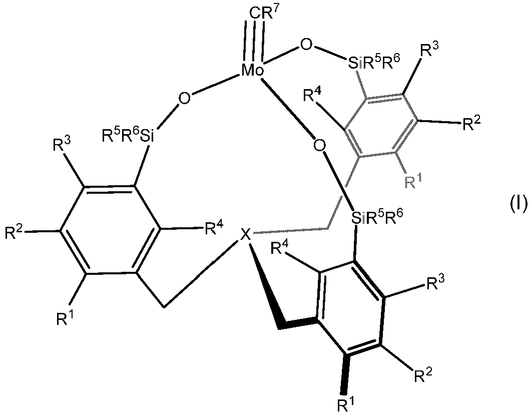 Wherein
Xis N,
R1, R2 and R3 are selected independently from H or C1-C6-alkyl,
R4 is H,
R5 and R6 may be equal or may be different from one another and represent C1-C12-alkyl or 6-18 membered aryl,
R7 represents C1-C12-alkyl or 6-18 membered aryl, which alkyl or aromatic ring structure may be substituted by one or more substituents selected from C1 to C6 alkyl, 6-14 membered aryl or heteroatoms.

On the basis of the metal-organic compound of Formula (I) before, the inventors have prepared a 1:1 adduct of said metal-organic compound of Formula (I) as defined before with an adduct-forming compound, selected from
- a nitrogen-containing 5 to 10 membered heteroaryl compound, said heteroaryl optionally being substituted by one or more C1- C6-alkyl, halogen, -CN, -CF₃, -N(C₁-C₆ alkyl)₂ or -NO₂ substituents, or
- PR^{P}₃ wherein R^{P} is independently selected from C1-C6-alkyl or 6-18 membered aryl, said alkyl or aryl optionally being substituted by one or more C1- C3-alkyl, halogen, - CN, -CF₃, -N(C₁-C₃ alkyl)₂ or -NO₂ substituents.

In a more defined embodiment, the invention refers to an adduct of a metal-organic compound of Formula (I) with an adduct-forming compound, wherein the adduct-forming compound is pyridine, optionally being substituted by one or more C1 -C6-alkyl, halogen, - CN, -CF₃, -N(C₁-C₆ alkyl)₂ or -NO₂ substituents with the proviso that at least one of the pyridine substituents on the 2 and 6 positions is H, the adduct with parent pyridine being represented by Formula (II): 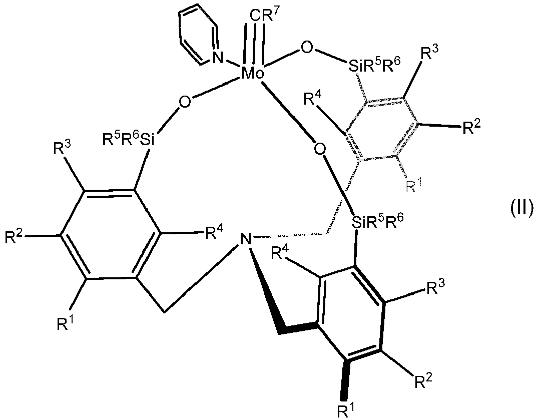 wherein R1 to R7 have the meaning as defined in any one of claims 1 to 4.

In the scope of the present invention, the process for preparing a metal-organic compound of the general Formula (I) is also disclosed: 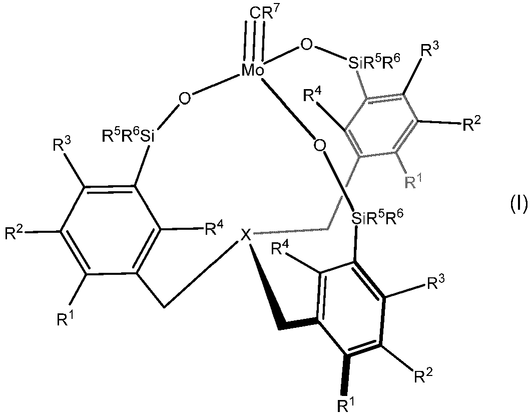 Wherein:
X represents N, CR^{x}, SiR^{x}, wherein R^{x} is selected from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more heteroatoms;
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl, or
(R1 and R2) or (R2 and R3) on the same phenyl ring form an aromatic or heteroaromatic ring structure conjugated with the phenyl ring and having a total of 10 to 18 carbon atoms in the ring structure wherein one to three ring carbon atoms may be replaced by nitrogen, which aromatic or heteroaromatic ring structure may be substituted by one or more heteroatoms,
R4 is selected from H, methyl or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, which alkyl, cycloalkyl, aromatic or heteroaromatic ring structure may be substituted by one or more heteroatoms,
R7 is selected from C1-C12-alkyl, C3-C12-cycloalkyl, 6-18 membered aryl, or 5-10 membered heteroaryl which alkyl, cycloalkyl, aromatic or heteroaromatic ring structure may be substituted by one or more substituents selected from C1 to C6 alkyl, 6-14 membered aryl or heteroatoms,
said process comprising the steps of reacting a molybdenum complex of the Formula (III) with a compound of the Formula (IV) to yield a compound of Formula (I). 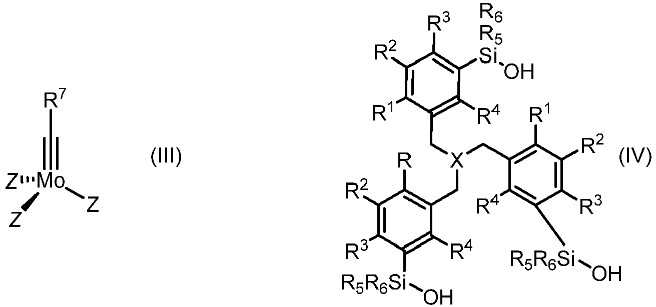 wherein Z represents C1-C6 alkyl, -O-C1-C6 alkyl, -N(C1-C6 alkyl)₂, -N(6-18 membered aryl)₂, or-N[(C1-C6 alkyl)(6-18 membered aryl)] and wherein X and R1 to R7 have the meaning as defined above for the metal organic compound of Formula (I).

In the inventive process, the choice of the solvent is not critical as long as the solvent is an aprotic non-polar organic solvent selected from diethylether, aromatic solvents such as benzene, toluene, aliphatic hydrocarbon solvents having 5 to 8 carbon atoms, such as pentane, hexane, or mixtures thereof. The reaction conditions are also not critical and the reaction is usually carried out at a temperature between 0°C and 80°C, preferably 10°C to 35°C, under ambient pressure and under an inert gas atmosphere.

The metal-organic compound of Formula (I) and the adducts thereof as defined before can be used for catalysing alkyne metathesis reactions. Therefore, the present invention is also directed to the use of the inventive metal-organic compound of Formula (I) or any adduct thereof as defined above as a catalyst for alkyne metathesis reactions.

In the context of the present invention, the meanings are as follows:
C1-C12 alkyl represents a straight or branched alkyl group having from 1 to 12 carbon atoms;
C1-C12 alkoxy represents a straight or branched chain alkoxy group having from 1 to 12 carbon atoms;
di-C1-C4 alkylamino represents an amino group having two identical or different straight-chain or branched alkyl substituents, each having from 1 to 4 carbon atoms;
C3-C12 cycloalkyl represents a monocyclic, saturated cycloalkyl group having 3 to 12 carbon atoms;
C3-C12 cycloalkyloxy represents a monocyclic saturated cycloalkyloxy group having 3 to 12 carbon atoms;
5- to 10-membered heteroaryl represents a mono- or optionally bicyclic aromatic heterocycle (heteroaromatic) having a total of 5 to 10 ring atoms, which contains up to three ring heteroatoms from the series N, O and/or S and is linked via a ring carbon atom or optionally via a ring nitrogen atom. Examples are: Furyl, pyrrolyl, thienyl, pyrazolyl, imidazolyl, thiazolyl, oxazolyl, isoxazolyl, isothiazolyl, triazolyl, oxadiazolyl, thiadiazolyl, pyridyl, pyrimidinyl, pyridazinyl, pyrazinyl, triazinyl, benzofuranyl, benzothienyl, benzimidazolyl, benzoxazolyl, benzothiazolyl, benzotriazolyl, indolyl, indazolyl, quinolinyl, isoquinolinyl, naphthyridinyl, quinazolinyl, quinoxalinyl, phthalazinyl, pyrazolo[3,4-b]pyridinyl.

A heteroatom or heterosubstituent as defined according to the invention can be selected from OH, halogen CN, NO₂, NO, NCO, -NCS, -SCN, SO₃H, a monohalogenomethyl group, a dihalogenomethyl group, a trihalogenomethyl group, CF(CF3)₂, SF₅, aliphatic, aromatic, heteroaromatic, primary, secondary, tertiary amine or ammonium bound through N atom, - O-alkyl (alkoxy), -O-aryl, -O-heteroaryl -O-SiR^{s}₃,-S-S-R^{s}, -S-R^{s}, -S(O)-R^{s}, -S(O)₂-R^{s}, - COOH, -CO₂-R^{s}, -BR^{s}₂, -PR^{s}₂,-OPR^{s}2, amide, bound through C or N atom, formyl group, - C(O)-R^{s}, -COOM, where M is a metal such as Li, Na, K, Cs, Ag. R^{s} may be, independently from each other, the same or different and is each an aliphatic, heteroaliphatic, aromatic or heteroaromatic group, each optionally being further substituted by one or more heterosubstituents, aliphatic, heteroaliphatic, aromatic or heteroaromatic groups; and/or optionally bridged by an -O- atom, represents a halogenide. Halogen in the context of the invention means fluorine, chlorine, bromine and iodine.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₆, C₃₋₄, C₄₋₆, C₄₋₆, and C₅₋₆.

Preferred alkyl groups in the context of the present invention are straight-chain or branched alkyl groups having 1 to 6 carbon atoms. Exemplary and preferred are: Methyl, ethyl, n-propyl, isopropyl, n-butyl, iso-butyl, sec-butyl, tert-butyl, 1-ethyl-propyl, n-pentyl and n-hexyl.

Preferred alkoxy groups in the context of the present invention are straight-chain or branched alkoxy groups having 1 to 6 carbon atoms. Exemplary and preferred are: Methoxy, ethoxy, n-propoxy, isopropoxy, n-butoxy, tert-butoxy, n-pentoxy and n-hexoxy.

Preferred cycloalkyl groups in the context of the present invention are cycloalkyl groups having 3 to 7 carbon atoms. Exemplary and preferred are: Cyclopropyl, cyclobutyl, cyclopentyl, cyclohexyl and cycloheptyl.

Preferred cycloalkyloxy groups in the context of the present invention are cycloalkyloxy groups having 3 to 7 carbon atoms. Exemplary and preferred are: Cyclopropyloxy, cyclobutyloxy, cyclopentyloxy, cyclohexyloxy and cycloheptyloxy.

Preferred di-C1-C4 alkylamino groups in the context of the present invention are the following di-C1-C4 alkylamino groups: N,N-dimethylamino, N,N-diethylamino, N-ethyl-N-methyl¬amino, N-methyl-N-n-propylamino, N-isopropyl-N-methyl¬amino, N-isopropyl-N-ethyl¬amino, N-isopropyl-N-n-propyl¬amino, N,N-diisopropylamino, N-n-butyl-N-methylamino and N-tert. - butyl-N-methylamino.

"Aryl" refers to a radical of a monocyclic or polycyclic (e.g., bicyclic or tricyclic) 4n+2 aromatic ring system (e.g., having 6, 10, or 14 pi electrons shared in a cyclic array) having 6-18 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, i.e., unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

6- to 18-membered aryl generally represents a mono-, bi- or tricyclic carbocyclic aromatic which in turn may bear from 0 to 5 substituents selected from the list: C1-C12-Alkyl, C1-C12-alkoxy, di-C1-C4-alkylamino, C3-C12-cycloalkyl, C3-C12-cycloalkyloxy, 6- to 18-membered aryl, 5- to 10-membered heteroaryl, halogen, cyano, nitro;

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

Preferred aryl groups in the context of the present invention are: Phenyl, naphthyl, anthryl, methylphenyl, dimethylphenyl, trimethylphenyl, methoxyphenyl, dimethoxyphenyl, trimethoxyphenyl, fluorophenyl, chlorophenyl, bromophenyl, iodophenyl, pentafluorophenyl, trifluoromethylphenyl, dimethylaminophenyl, C1-C12 alkoxycarbonylphenyl.

"Heteroaryl" refers to a radical of a 5-10 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom (*e.g*., 2-indolyl) or the ring that does not contain a heteroatom (*e.g*., 5-indolyl).

5- to 10-membered heteroaryl generally represents a mono- or optionally bicyclic aromatic heterocycle (heteroaromatic) having a total of 5 to 10 ring atoms, which contains up to three ring heteroatoms from the series N, O and/or S and is linked via a ring carbon atom or optionally via a ring nitrogen atom. Examples are: Furyl, pyrrolyl, thienyl, pyrazolyl, imidazolyl, thiazolyl, oxazolyl, isoxazolyl, isothiazolyl, triazolyl, oxadiazolyl, thiadiazolyl, pyridyl, pyrimidinyl, pyridazinyl, pyrazinyl, triazinyl, benzofuranyl, benzothienyl, benzimidazolyl, benzoxazolyl, benzothiazolyl, benzotriazolyl, indolyl, indazolyl, quinolinyl, isoquinolinyl, naphthyridinyl, quinazolinyl, quinoxalinyl, phthalazinyl, pyrazolo[3,4-b]pyridinyl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

Preferred heteroaryl groups in the context of the present invention are mono- or optionally bicyclic 5- to 10-membered heteroaryl groups containing up to two heteroatoms from the series N, O and/or S. Particularly preferred are monocyclic 5- or 6-membered heteroaryl groups with up to two heteroatoms from the series N, O and/or S such as, for example, furyl, thienyl, thiazolyl, oxazolyl, isothiazolyl, isoxazolyl, pyrazolyl, imidazolyl, pyridyl, pyrimidinyl, pyridazinyl, pyrazinyl.

Examples of preferred silanolate bridges of the general type R⁵R⁶SiO- in the context of the present invention are: Diphenyl silanolate, dialkyl silanolate, di(tert-butyl)silanolate, phenyl methyl silanolate, phenyl(tert-butyl)silanolate, di(methoxyphenyl)silanolate, di(dimethoxyphenyl)silanolate, di(dimethylphenyl)-silanolate, di(trimethylphenyl)silanolate, di(dimethylaminophenyl)silanolate, di(fluorophenyl)silanolate, di(difluorophenyl)silanolate, di(pentafluorophenyl)silanolate, di(3,5-di-tert-butyl-6-methoxyphenyl)silanolate, di(1-naphthyl)silanolate, di(2-furyl)silanolate, di(2-thienyl)silanolate.

Amongst the possible candidates for adduct formation (amines, pyridines, quinolones, oxazoles, thiazoles, thioethers, nitriles, phosphines, phosphites etc.), substituted pyridines were deemed particularly adequate because many of them differing in donor character and steric demand are commercially available.

The present invention is further illustrated by Experimental Part and the attached Figures. In the Figures, the following is illustrated:

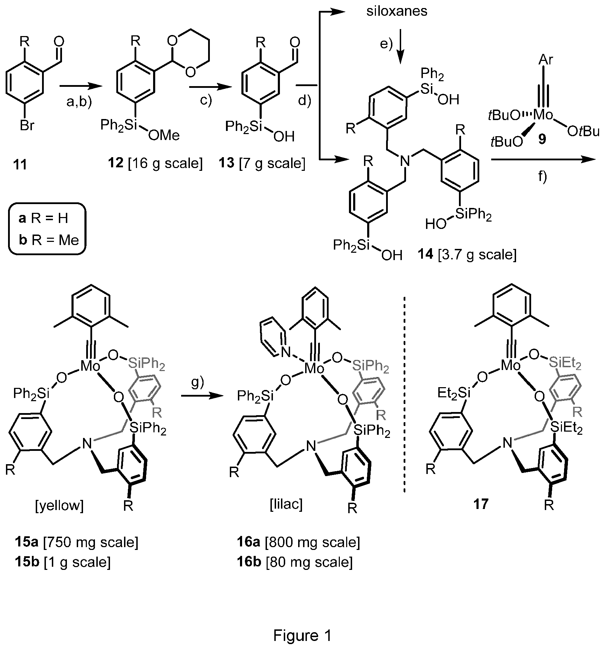

- Figure 1:: Reagents and conditions of catalyst synthesis and complex synthesis
(a) 1,3-propanediol, *p*TsOH (3 mol%), toluene, reflux, 81% (R = H), 72% (R = Me)
(b) (i) *i*-PrMgBr (0.4 equiv.), n-BuLi (0.8 equiv.), THF, 0°C; (ii) Ph₂Si(OMe)₂, 0°C → RT, 85% (R = H), 70% (R = Me);
(c) HCl (6 M), THF, 0°C, 92% (R = H), 94% (R = Me);
(d) NH₄OAc, NaBH(OAc)₃, THF;
(e) NaOH (2 M), THF, 60% (over two steps, R = H), 65% (over two steps, R = Me)
(f) **9**, toluene, 94% (R = H), 98% (R = Me);
(g) pyridine, CH₂Cl₂, 88% (R = H), 77% (R = Me);
The indicated scales refer to the single largest batch for R = Me
- Figure 2:: Photograph of a sample of crystalline **16b**; well resolved aromatic region of the ¹H NMR spectrum ([D₈]-toluene, 253 K) of this sample after storage in air without any precautions at ambient temperature for 8 months, showing only trace impurities caused by hydrolysis

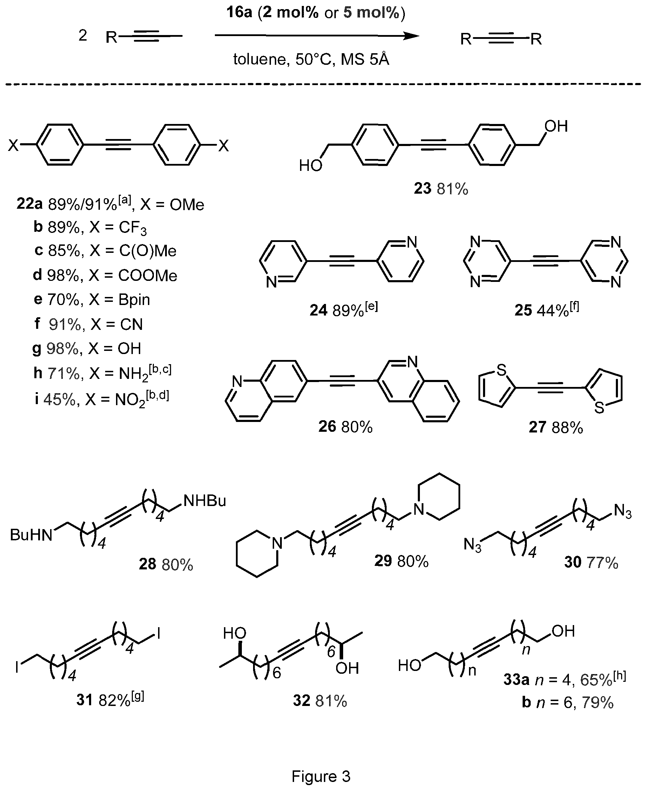

- Figure 3:: Homo-metathesis reactions of functionalized substrates
[a] 89% at 50°C, 91% at RT;
[b] using the free complex **17** instead of adduct **16a**;
[c] at 110°C;
[d] with 7 mol% of catalyst at 110°C;
[e] at 90°C;
[f] at 100°C;
[g] with added BPh₃ at room temperature;
[h] with MS 4Å (instead of 5Å) ; the chosen catalyst loading is color-coded
- Figure 4:: Macrocycles by ring closing alkyne metathesis
All reactions were performed in toluene (2 mM) in the presence of MS 5Å using the indicated catalyst; all substrates were carrying methyl caps on the triple bonds; [a] a modified "assisted" work up was necessary to obtain pure samples,
- Figure 5:: Advanced target molecules by ring closing alkyne metathesis
(a) **16a** (5 mol%), 60°C, toluene, MS 5Å, 81%;
(b) **16a** (5 mol%), 50°C, toluene, MS 5Å, 65%;
(c) **16a** (10 mol%), 60°C, toluene, MS 5Å, 94%;
(d) **16a** (30 mol%), 110°C, toluene, MS 5Å, 71%;
(e) **16a** (30 mol%), 110°C, toluene, MS 5Å, 81%.

As illustrated by a representative example in Figure 1, the preparation of the envisaged ligands proved straightforward on multigram scale. The commercial bromobenzaldehydes **11** (R = H, Me) were transformed into the corresponding acetals prior to metal/halogen exchange on treatment with *i*-PrMgBr/*n*-BuLi. The resulting organometallic species were quenched with Ph₂Si(OMe)₂ and the siloxanes **12** thus formed treated with aq. HCl for concomitant release of the Si-OH group and the aldehyde. Subsequent reductive amination of **13** furnished the targeted tris-silanolates **14**. However, this step is accompanied by variable degrees of inter- and/or intramolecular siloxane formation. Stirring of the oligomeric fraction with aq. NaOH in THF sufficed to rectify the issue and to regenerate the desired monomer, thus making the targeted ligands **14** available on scale in good overall yield from cheap starting materials.

In the solid state, compound **14b** orients all three silanol groups "upward/inward" likely as the result of mutual intramolecular hydrogen bonding between the -OH groups. These interactions entail a preorganization of the ligand scaffold that likely persists in an aprotic medium; chelate complex formation should hence be favorable. In fact, stirring of a solution of **14** and **9** in toluene furnished the desired complexes **15** in excellent yields as yellow solid materials. The formation of oligomeric complexes does not interfere to any noticeable degree and washing of the crude product with pentane usually suffices to remove traces of residual substrates, thus rendering the isolation of **15** in analytically pure form facile. A minor procedural adjustment also brought the ethyl variant **17** into reach (see the Experimental Part). Other ligands of this type carrying yet other substituents on silicon and/or on the aryl rings of the backbone can be prepared analogously.

Addition of a slight excess of pyridine to a yellow solution of **15b** in CH₂Cl₂ at ambient temperature causes a color change to deep lilac. Evaporation of the solvent and washing of the residue with pentane to remove excess pyridine affords adduct **16b** as a lilac powder that can be recrystallized, for eample, from CH₂Cl₂/pentane to give purple single crystals suitable for X-ray diffraction. The structure in the solid state confirms the tight binding of the external pyridine ligand (Mo1-N2 2.255(1) Å), whereas the N-atom of the tris-benzylic linker is off the metal center (Mo1/N1 5.38 Å); it has solely a geometric function but exerts no electronic effect.

A number of pyridine derivatives differing in steric demand and donor ability was screened in the quest for a potentially even better stabilizing ligand. Whereas 2,6-dimethylpyridine does not bind at all to **15**, the adducts derived from 3-bromopyridine and 3,5-dibromopyridine proved labile (the external ligand is partly lost upon washing of the crude material with pentane). The more electron rich 4-pyrrolidinopyridine led to a stable adduct that is hardly prone to decomplexation at room temperature. The choice of the pyridine hence allows the stability of the adducts to be tuned over a wide range. For practical purposes, cheap pyridine itself is actually a good comprise: the resulting adduct **16** is storable at the benchtop or in a freezer for extended periods of time and can be weighed in air, while solutions in toluene show excellent catalytic activity and allow a multitude of challenging alkyne metathesis reactions to be carried out at or slightly above room temperature without the need for separate preactivation of the adduct.

With a half-lifetime on the order of days, even the pyridine-free complex **15b** shows an appreciable robustness when kept in air as a microcrystalline powder; it can be weighed and handled without particular precautions, although long-term storage requires inert conditions.

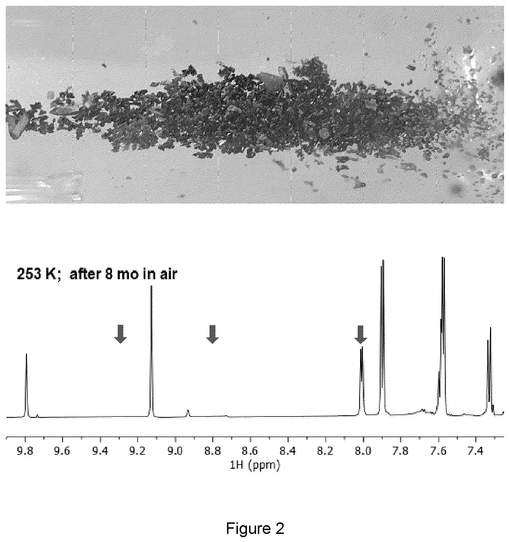

Complexation to pyridine increases the stability to a significant extent. Hardly any signs of degradation were detected by ¹H NMR when adduct **16b** was stored in crystalline form in air at ambient temperature for up to eight months as shown in Figure 2). When stored in air as a powder (rather than in crystalline form) at ambient temperature, hydrolysis becomes more prominent with time. Gratifyingly, however, even the integrity of powder samples is easy to maintain, simply upon storage in a desiccator or, alternatively, in a screw-capped vial in a freezer. Under these conditions, powder samples stay intact for many months as proven by NMR as well as elemental analysis; importantly, all catalytic test reactions occurred with unchanged rate and efficiency. We hence conclude that pyridine adducts **16** are not fully inert towards moisture and will eventually hydrolyze, but long-term storage is possible outside a glovebox under conditions that are easy to realize (for example in a desiccator and/or freezer). Such high stability and procedural simplicity is arguably unmatched for the time being. Adducts **16** are hence deemed enabling and practical tools adequate for use even by non-experts.

When dissolved in an inert solvent such as toluene, the pyridine adducts **16** exhibit appreciable catalytic activity at ambient temperature by virtue of the spontaneous release of **15** as monitored by NMR; separate activation is not necessary. The metathesis reaction of 1-methoxy-4-(prop-1-yn-1-yl)benzene reaches the equilibrium composition in ≈60 min at 25°C when catalyzed by the pyridine adduct **16a** (5 mol%) in [D₈]-toluene in the absence of molecular sieves. As expected, raising the temperature results in massive rate acceleration.

Although these results prove that the air-persistent pyridine adduct **16** itself is a fully competent catalyst that does not need any pre-activation other than dissolution in an inert solvent, it is possible to promote the reaction, if one so desires. Added BPh₃ (1 equiv. relative to **16**) scavenges the pyridine and hence largely shifts the equilibrium between **15** and **16** to the side of the free catalyst; this comes along with a characteristic color change of the solution from lilac to yellow. Under these conditions, the test reaction proceeded within minutes at ambient temperature. While promotors other than BPh₃ can be envisaged, this crystalline compound is deemed a good choice for the ease of handling (although not fully air-stable, it was used without any precautions) and the benign character; it retains the high affinity of trivalent boron species to N-donors but exhibits a tempered Lewis-acidity and hence likely poses little risk in advanced applications.

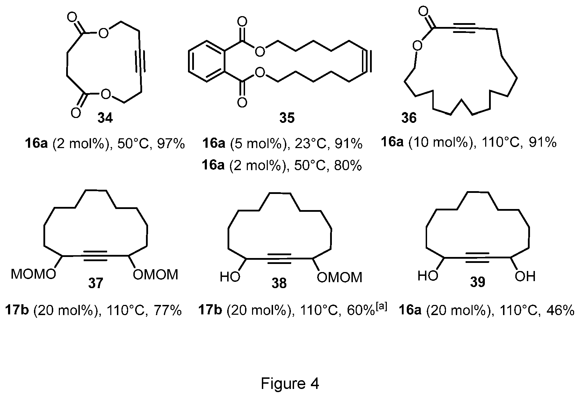

The chelate catalysts of the new series exhibit a favorable application profile. Most reactions can be performed with ≤ 2 mol% of the bench-stable adduct **16a** as the catalyst in toluene in the presence of MS 5Å as scavenger for the released 2-butyne; however, higher catalyst loadings (usually 5 mol %) were employed for substrates carrying protic substituents. The catalysts are operative in a temperature range between at least -20°C and +130°C, preferentially they are used between ambient temperature and +70°C; the optimal reaction temperature can depend on the chosen substrate. In many cases, the choice of temperature is not critical and has little effect on the yield of the metathesis product; this aspect is illustrated by the formation of product **22a** (Figure 4) as well as product **35** (Figure 4) at different reaction temperatures.

A series of homo-metathesis reactions confirmed the broad functional group tolerance of **16** (Figure 4). A few entries deserve particular comment. Protic groups may eventually react with the complex, replace the silanolates and, in doing so, spoil catalytic activity; therefore, they had marked an important limitation for alkyne metathesis in the past and were one of the main reasons for the development of catalysts harnessing the chelate effect (J. Am. Chem. Soc. 2021, 143 (38), 15538-15555). Gratifyingly, the newly invented and user-friendly catalysts **16** prove adequate. They operate in the presence of primary and secondary aliphatic, benzylic and propargylic alcohols, a phenolic -OH, the -NH₂ substituent of an aniline, as well as the -NH group of a secondary amine. As mentioned above, however, a loading of 5 mol% was used in these cases to ensure full conversion.

The fact that binding of external pyridine to the active molybdenum alkylidyne is reversible even at ambient temperature in toluene solution forecasts an excellent compatibility of the new catalysts with various donor sites. Indeed, catalysts **16** comprising an early transition metal center in the highest possible oxidation state are compatible with different heterocycles including, but not limited to: pyridine, pyrimidine, quinoline, thiophene, thiazole, oxazole, N-alkyl piperidine; catalysts **16** are also compatible with Lewis-basic sites including, but not limited to: aliphatic secondary and tertiary amine, secondary and tertiary alkyl(aryl)amines, anilines, phosphines, thioethers, nitriles. Higher reaction temperatures were necessary only in those cases in which the functional group renders the triple bond to be metathesized electron-deficient. Comparison with [(*t*BuO)₃W≡CCMe₃] as the prototype of a catalytically active alkylidyne complex clearly illustrates the advance, as this catalyst had previously failed to convert substrates comprising a thiazole ring or even a pyridine of reduced basicity.

Additional functional groups that were found to be compatible with **15-17** include, but are not limited to: terminal and internal alkenes, acetals, aldols, alkyl, alkenyl and aryl halides, alkyl azides, boronate esters, carbamates, carbonates, 1,2-dibromoalkenes, ethers, epoxides, esters, ketones, nitriles, -NO₂, -CF₃, silyl ethers.

A number of ring closing alkyne metathesis (RCAM) reactions catalyzed by **16** or **17** gave excellent results (Figure 4). As expected, macrocycles **34** and **35** were obtained in high yields. The reactions can be performed at room temperature or at elevated temperatures , as deemed desirable. Product **36** constitutes a more stringent test: for their low reactivity, ynoates and related substrates had been beyond the scope of the early catalyst generations including [(*t*BuO)₃W≡CCMe₃] as the historic landmark in the field. As far as we know, only molybdenum alkylidynes with silanolates have so far been found capable of forming macrocyclic ynoates by RCAM; the new user-friendly variant **16** retains this ability.

Cycloalkynes such as **37-39** carrying -OR substituents on both propargylic positions are demanding targets (Figure 4). Only few such examples made by RCAM are known in the literature, again relying on molybdenum alkylidynes ligated to silanolates. It is therefore noteworthy that **16** and **17** are able to form products of this type. It turned out that product **38** and the tris-silanolate ligand hydrolyzed off the catalyst upon work-up co-elute during flash chromatography, thus making product purification challenging. The N-atom in the ligand backbone proved handy to address the issue. Thus, the organic phase was washed with aqueous HCl before the solvent was evaporated and the residue purified by flash chromatography; under these conditions, the ligand was quantitatively removed. Although only sufficiently acid-stable products can be processed in this way, such a "ligand-assisted" work-up is another asset of the new catalyst family in practical terms.

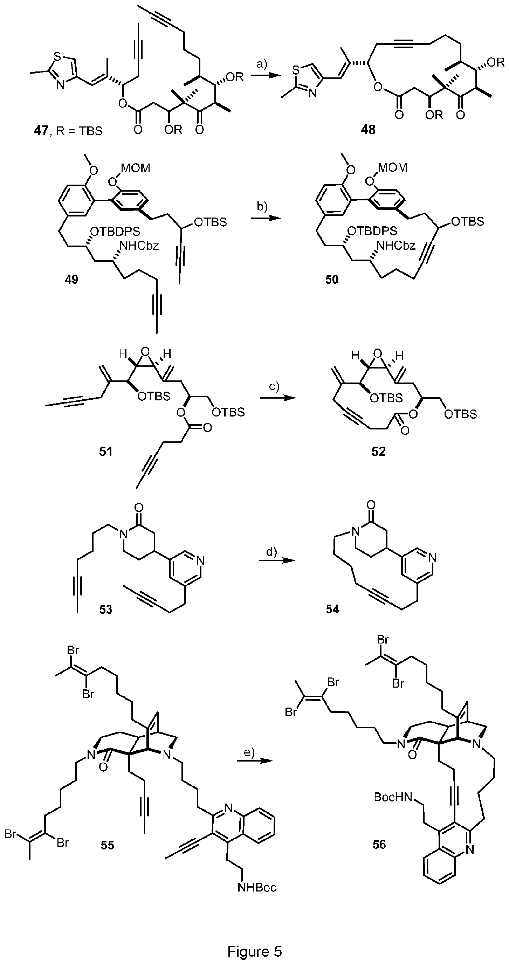

The inventors used a selection of advanced intermediates of previous natural product synthesis campaigns pursued in the inventor's laboratory (Figure 5). The highly functionalized cycloalkyne **48** as precursor for the anticancer agent epothilone C can be readily formed with the aid of the new bench stable pyridine adduct **16**. The successful formation of product **50**, which had served as key intermediate in the total synthesis of the alkaloid lythranidine. Taken together, these examples show the compatibility of the new bench-stable adduct **16** with many different polar and apolar functional groups, even when presented as dense arrays.

Alkyne metathesis is orthogonal to alkene metathesis in that molybdenum alkylidynes leave all types of double bonds untouched. This favorable chemoselectivity surfaces in the epothilone case (**47→48**) but is more prominently manifested in the high-yielding formation of **52** with the help of the bench stable pyridine adduct **16**. This product belongs to the amphidinolide V series: one of the two exo-methylene groups decorating the macrocycle is part of a vinyl-epoxide and hence of a quite reactive functional group, whereas the other one carries a potential leaving group at the allylic position.

The compatibility of the new catalysts with different Lewis-basic groups is further illustrated by the formation of product **54**, the key precursor en route to the marine pyridinium alkaloid *epi*-tetradehydro-halicyclamine B; in this case, the macrocyclization has to be performed in the presence of an unhindered pyridine. In the inventor's initial report, this reaction had been achieved with the aid of a "canopy" catalyst that is sensitive to air (Angew. Chem. Int. Ed. 2022, 61, e202209651). It is now shown that the much more user-friendly bench-stable adduct **16a** is similarly effective.

The high-yielding formation of product **56** comprising the basal macrocycle of nominal njaoamine I, a member of a family of marine alkaloids endowed with cytotoxic properties, is particularly instructive. Although elevated temperatures were necessary for the reaction to proceed, the presence of a tertiary amine as well as a quinoline in proximity to the reacting triple bonds arguably poses a challenge for almost any transition metal reagent or catalyst. The excellent yield achieved with the aid of **16a** as catalyst is hence remarkable. In terms of efficiency, the new air-stable adduct **16a** is on bar with the air-sensitive molybdenum alkylidynes used in our original study (J. Am. Chem. Soc. 2021, 143 (35), 14402-14414). Equal performance combined with significantly improved user-friendliness marks an important advance in the field of alkyne metathesis in general.

The present invention is further illustrated by the Experimental Part as follows.

### Experimental Part

### General

Unless stated otherwise, all reactions were carried out under argon in flame-dried glassware following standard Schlenk techniques. The solvents were purified by distillation over the drying agents indicated and were transferred under argon: tetrahydrofuran (magnesium/anthracene), toluene (NaAlEt₄), benzene (CaH₂), dichloromethane (CaH₂), 1,2-dimethoxyethane (CaH₂), acetonitrile (CaH₂), diethyl ether (Na/K), and *n*-pentane (Na/K). Deuterated solvents were stored over molecular sieves (3 Å) for a minimum of five days prior to use.

The molecular sieves and Celite^{®} used in this investigation were dried for 24 h at 150 °C (sand bath) under vacuum (10⁻³ mbar) prior to use and were stored and transferred under argon atmosphere.

Flash chromatography was carried out with Merck silica gel, type 9385 (230 - 400 mesh, 60 Å pore diameter).

All commercially available compounds (abcr, Lancaster, Aldrich, TCI) were used as received, unless stated otherwise. Substrates for alkyne metathesis reactions were prepared as described in the literature. Starting materials for the intermediates of natural product syntheses were graciously provided by Dr. D. Dalling, Dr. C. Mathes, Dr. O. Larionov, Dr. K. Gebauer and Dr. S. Spohr.

MS (El): Finnigan MAT 8200 (70 eV), ESI-MS: ESQ3000 (Bruker), accurate mass determinations: Bruker APEX III FT-MS (7 T magnet) or Mat 95 (Finnigan). All values are given in mass units per elementary charge (*m*/*z*).

### Experimental Procedures

### Molybdenum Precursor Complexes

### Complex [Li·(Et₂O)_{1/2}][Mo(CO)₅(COAr)] (S1, Ar = 2,6-di(methyl)phenyl)

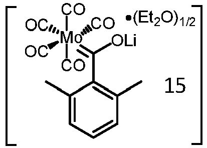

A 250 mL, three-necked, round bottom flask equipped with a 50 mL dropping funnel and connected to a vacuum/argon manifold was flame dried. The flask was charged with 2-bromo-1,3-dimethylbenzene (3.28 mL, 24.1 mmol) and diethyl ether (60 mL). The solution was cooled to -40 °C before a solution of *tert*-butyllithium (1.6 M in *n*-pentane, 30.1 mL, 48.3 mmol) was added *via* the dropping funnel over 10 min. Stirring was continued for 3 h at -20 °C.

A 500 mL, three-necked, round bottom flask equipped with a 100 mL dropping funnel and connected to a vacuum/argon manifold was flame-dried. The flask was charged with molybdenum hexacarbonyl (6.50 g, 24.1 mmol) and diethyl ether (60 mL), and the resulting solution was cooled to 0 °C. The solution of the lithiated arene was cooled to -78 °C, transferred with argon overpressure into the dropping funnel *via* a low density polyethylene (LDPE) tube, and then rapidly added to the cold solution of the molybdenum hexacarbonyl. Once the addition was complete, the resulting yellow-orange mixture was stirred at ambient temperature for 4 h. The dropping funnel was replaced by a glass stopper and a solvent trap was connected to the vacuum/argon manifold. The solvents and all volatile materials were evaporated under reduced pressure (10⁻³ mbar). The resulting orange solid was suspended in dichloromethane (4 × 20 mL) and filtered through an argon filter frit packed with a Celite^{®} pad mounted onto a flame dried, 250 mL, three-necked flask connected to a vacuum/argon manifold. The resulting filtrate was concentrated *in vacuo* (10⁻³ mbar) until ca. 10 mL of the solution remained.

A flame dried, 1 L, three-necked flask equipped with an argon manifold was charged with *n*-pentane (700 mL) and the concentrated solution was added dropwise with vigorous stirring. The product precipitated as a microcrystalline solid, which was collected by filtration using an argon filter frit mounted onto a flame dried, 2 L two-necked flask connected to a vacuum/argon manifold. The product was washed with *n*-pentane (3 × 20 mL) and dried under high vacuum (10⁻³ mbar) to give the desired complex as an diethyl ether adduct (2:1 ratio). Pale yellow microcrystalline solid material (8.76 g, 95%). HRMS (ESI) calc. for C₁₄H₉O₆MoLi [M - Et₂O - Li]⁻: 370.94587; found: 370.94613.The spectroscopic data was found to be in agreement with the data reported in the literature.

### [Mo(≡CAr)Br₃(dme)] (S2, Ar = 2,6-di(methyl)phenyl)

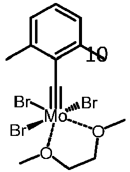

A 500 mL, three-necked, round bottom flask equipped with an internal thermometer and connected to a vacuum/argon manifold was flame dried. The flask was charged with [Li·(Et₂O)_{1/2}][Mo(CO)₅(COAr)] (S1) (4.37 g, 10.6 mmol) and dichloromethane (60 mL). The resulting orange mixture was cooled to -78 °C and stirred for 15 min before a solution of oxalyl bromide (1.09 mL, 11.6 mmol) in dichloromethane (5 mL) was added dropwise over 5 min, causing a color change to purple. Once the addition was complete, stirring was continued for 15 min at -78 °C. The mixture was then allowed to warm to -40 °C. Upon reaching this temperature a rapid color change to light brown was observed and the mixture was quickly cooled to -78 °C again. Stirring was discontinued to allow most of the precipitate to settle.

In parallel, a 1 L, three-necked, jacketed cooling flask equipped with a 25 mL pressure-equalizing dropping funnel and a jacketed cooling argon filter frit (diameter: 4.5 cm; height: 20 cm; porosity: 3) containing a pad of Celite^{®} (5 cm) was connected to a vacuum/argon manifold and flame dried. The Celite^{®} in the jacketed filter frit was slurried with dichloromethane and packed tightly before the frit was cooled to -78 °C using a cryostat. This flask was charged with 1, 2-dimethoxyethane (5.50 mL, 52.9 mmol), which was also cooled to -78 °C.

The cold light brown reaction mixture was transferred with argon overpressure *via* a LDPE tube in portions to the frit. Upon contact of the bright yellow filtrate with the 1,2-dimethoxyethane in the 1 L receiving flask, a color change to dark red was observed. The mixture was stirred for 10 min at -78 °C before a solution of bromine (0.51 mL, 10.0 mmol) in dichloromethane (5 mL) was added dropwise over a period of 15 min *via* the dropping funnel. Once the addition was complete, the mixture was stirred for 15 min at -78 °C. The resulting brown-orange mixture was allowed to reach ambient temperature over the course of 1 h, during which time the color intensifies. Stirring was continued for 1 h at ambient temperature before the dropping funnel and the filter frit were replaced by glass stoppers. The brown reaction mixture was filtered through an argon filter frit with a Celite^{®} pad mounted onto a flame dried, 500 mL, two-necked flask connected to a vacuum/argon manifold. The frit was replaced by a stopper and the solvents were removed from the filtrate *in vacuo* to give the title complex as a rust colored solid (2.30 g, 41%). Impurities could be removed by washing the solid with diethyl ether. The spectroscopic data was found to be in agreement with the data reported in the literature.

### [Mo(≡CAr)(OtBu)₃] (9, Ar = 2,6-di(methyl)phenyl)

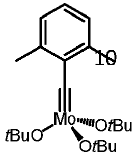

A 250 mL Schlenk flask was charged with [Mo(≡CAr)Br₃(dme)] (Ar = 2,6-di(methyl)phenyl) S2 (2.05 g, 3.79 mmol,) and tetrahydrofuran (23 mL). A solution of sodium tert-butoxide (1.07 g, 11.2 mmol) in tetrahydrofuran (7 mL) was added dropwise *via* syringe at ambient temperature and stirring was continued overnight before the solvent was removed *in vacuo* to obtain a dark brown solid material. The residue was suspended in n-pentane (4 × 20 mL) and the suspension was filtered through a Celite^{®} pad in an argon filter frit mounted onto a 250 mL, two-necked flask connected to a vacuum/argon manifold. The filter frit was replaced by a stopper and the filtrate was concentrated *in vacuo* (10⁻³ mbar) until a total volume of ca. 4 mL remained. The solution was cooled to -40 °C, causing crystallization of the title complex over a course of 4 h. Removal of the supernatant and drying of the residue under high vacuum (10⁻³ mbar) gave the desired complex as a brown powder (1.31 g, 3.04 mmol, 80%). The spectroscopic data was found to be in agreement with the data reported in the literature.

### The Ligands

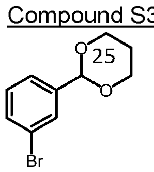

A 100 mL, two-necked round bottom flask equipped with a Dean-Stark apparatus (reflux condenser and distilling trap filled with toluene) and connected to a vacuum/argon manifold was charged with 5-bromobenzaldehyde (6.85 g, 37.0 mmol), toluene (50 mL) and propan-1,3-diol (2.94 mL, 40.7 mmol). *p*-Toluenesulfonic acid (211 mg, 1.11 mmol) was added and the solution was stirred at reflux temperature overnight. After cooling to ambient temperature, the organic layer was washed with an aqueous solution of sodium bicarbonate (2 × 50 mL) and brine (1 × 50 mL). The organic phase was dried over magnesium sulfate and filtered, and the solvents were evaporated. The residue was purified by flash chromatography on silica (hexane/ethyl acetate, 19:1 to 9:1) to give the title compound as a yellow liquid (7.25 g, 81%). The spectroscopic data was found to be in agreement with the data reported in the literature.

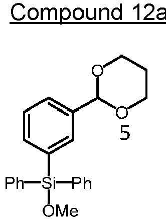

A 100 mL Schlenk flask was charged with compound S3 (7.25 g, 29.8 mmol) and tetrahydrofuran (30 mL). The solution was cooled to 0 °C before a solution of isopropylmagesium chloride lithium chloride complex (1.25 M in tetrahydrofuran, 9.18 mL, 11.9 mmol) was added dropwise. After stirring for 5 min, a solution of n-butyllithium (1.6 M in hexane, 14.6 mL, 23.9 mmol) was added dropwise and the resulting grey suspension was stirred for 1 h at 0 °C. A 250 mL, two-necked round bottom flask equipped with a pressure equilibrating dropping funnel and connected to a vacuum/argon manifold was charged with dimethoxydiphenylsilane (13.5 mL, 59.6 mmol) and tetrahydrofuran (25 mL). The resulting solution was cooled to 0 °C. The Grignard reagent was transferred into the dropping funnel with a LDPE tube and argon overpressure and then slowly added to the dimethoxydiphenylsilane solution at 0°C. Once the addition was complete, the mixture was allowed to reach ambient temperature and stirring was continued overnight. The reaction was carefully quenched by the addition of an saturated aqueous solution of ammonium chloride (100 mL). The layers were separated and the aqueous phase was extracted with ethyl acetate (3 × 75 mL). The combined organic layers were dried over magnesium sulfate and filtered, and the solvents were evaporated. The residue was purified by flash chromatography on silica (hexane/ethyl acetate, 9:1) to give the title compound as a colorless oil (9.58 g, 85%). HRMS (EI) calc. for C₂₃H₂₄O₃SiNa [M + Na]⁺: 399.13869; found: 399.13871.

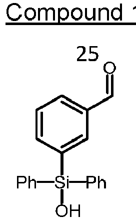

A 500 mL, one-neck round bottom flask equipped with a 100 mL dropping funnel was charged in air with compound 12a (9.58 g, 24.53 mmol) and tetrahydrofuran (245 mL). The solution was cooled to 0 °C before hydrochloric acid (6 nn in water, 62 mL, 0.37 mol) was added *via* the dropping funnel over 10 min. The mixture was warmed to ambient temperature and stirred for 1 h. The solution was carefully neutralized with an aqueous solution of sodium bicarbonate and extracted with dichloromethane (3 × 150 mL). The combined organic layers were dried over sodium sulfate and filtered, and the solvents were evaporated. The residue was purified by flash chromatography on silica (toluene/ethyl acetate, 10:1) to give the title compound as a colorless solid (6.88 g, 92%). HRMS (ESI) calc. for C₁₉H₁₆O₂SiNa [M + Na]⁺: 327.08118; found: 327.08071.

### Ligand 14a

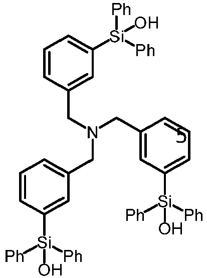

A 500 mL, two-necked round bottom flask connected to a vacuum/argon manifold was charged with compound 13a (8.20 g, 26.9 mmol), tetrahydrofuran (270 mL) and ammonium acetate (642 mg, 8.33 mmol). Sodium trisacetoxyborohydride (9.10 g, 42.9 mmol) was added in portions and the resulting suspension was stirred for 24 h. The solution was neutralized with an aqueous solution of sodium bicarbonate and extracted with ethyl acetate (3 × 200 mL). The combined organic layers were dried over magnesium sulfate and filtered, and the solvents were evaporated. The residue was purified by flash chromatography on silica (dichloromethane/methanol, 99:1 to 98:2) to give a mixture of siloxane dimers/oligomers and ligand 14a as a colorless powder. A 500 mL, one-necked flask was charged in air with this crude material and tetrahydrofuran (400 mL). An aqueous solution of sodium hydroxide (2 M, 400 mL) was added and the mixture was stirred overnight. The layers were separated and the aqueous layer was extracted with ethyl acetate (3 × 300 mL). The combined organic phases were washed with brine (300 mL), dried over magnesium sulfate and filtered, and the solvents were evaporated to give the title compound as a colorless powder (4.75 g, 60%). HRMS (ESI) calc. for C₅₇H₅₀NO₃Si₃ [M - H]⁻: 880.31041; found: 880.31091.

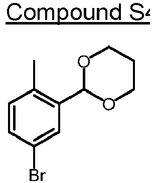

A 250 mL, two-necked round bottom flask equipped with a Dean-Stark apparatus (reflux condenser and distilling trap which was filled with toluene) and an argon manifold was charged with 5-bromo-2-methylbenzaldehyde (11b, 15.6 g, 78.4 mmol), toluene (100 mL), and propan-1,3-diol (6.23 mL, 86.2 mmol). *p*-Toluenesulfonic acid (447 mg, 2.35 mmol) was added and the solution was stirred at reflux temperature overnight. After cooling to ambient temperature, the organic layer was washed with an aqueous solution of sodium bicarbonate (2 × 75 mL) and brine (1 × 100 mL). The organic layer was dried over magnesium sulfate and filtered, and the solvents were evaporated. The residue was purified by flash chromatography on silica (hexane/ethyl acetate, 9:1) to give the title compound as a yellow liquid (14.9 g, 72%). HRMS (EI) calc. for C₁₁H₁₃O₂Br [M]⁻: 256.00954; found: 256.00935.

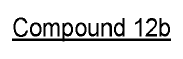

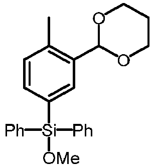

A 100 mL Schlenk flask was charged with compound S4 (14.9 g, 56.7 mmol) and tetrahydrofuran (20 mL). The solution was cooled to 0 °C before a solution of isopropylmagesium chloride lithium chloride complex (1.25 M in tetrahydrofuran 18.5 mL, 23.1 mmol) was added dropwise. After stirring for 5 min, a solution of n-butyllithium (1.6 M in hexane, 28.4 mL, 46.3 mmol) was added dropwise and the resulting grey suspension was stirred for 1 h at 0 °C. A 250 mL, two-necked flask equipped with a pressure equilibrating dropping funnel and connected to a vacuum/argon manifold was charged with dimethoxydiphenylsilane (26.2 mL, 116 mmol) and tetrahydrofuran (50 mL). The resulting solution was cooled to 0 °C. The Grignard reagent was transferred into the dropping funnel with a LDPE tube and argon overpressure and was then slowly added to the cooled dimethoxydiphenylsilane solution. Once the addition was complete, the mixture was allowed to warm to ambient temperature and stirring was continued overnight. The reaction was carefully quenched with a saturated aqueous solution of ammonium chloride (50 mL). The layers were separated and the aqueous phase was extracted with ethyl acetate (3 × 30 mL). The combined organic layers were dried over magnesium sulfate and filtered, and the solvents were evaporated. The residue was purified by flash chromatography on silica (hexane/ethyl acetate, 19:1) to give the title compound as a colorless oil (15.8 g, 70%). HRMS (EI) calc. for C₂₄H₂₆O₃Si [M]⁻ : 390.16457; found: 390.16510.

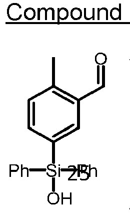

A 500 mL, one-neck flask equipped with a 100 mL dropping funnel was charged in air with compound 12b (8.72 g, 22.3 mmol) and tetrahydrofuran (220 mL). The solution was cooled to 0 °C and hydrochloric acid (6.0 M in water, 57 mL, 0.34 mol) was added *via* the dropping funnel over 10 min. The mixture was warmed to ambient temperature and stirred for 1 h. The solution was carefully neutralized with a saturated aqueous solution of sodium bicarbonate and the aqueous layer was extracted with dichloromethane (3 × 100 mL). The combined organic phases were dried over sodium sulfate and filtered, and the solvents were evaporated. The residue was purified by flash chromatography on silica (toluene/ethyl acetate, 14:1) to give the title compound as a colorless liquid (6.68 g, 94%). HRMS (EI) calc. for C₂₀H₁₈O₂SiNa [M + Na]⁺: 341.09704; found: 341.09683.

### Ligand 14b

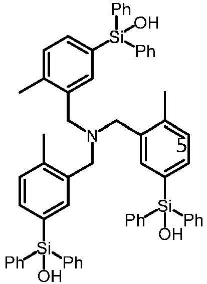

A 250 mL, two-necked round bottom flask connected to a vacuum/argon manifold was charged with compound 13b (5.00 g, 15.7 mmol), tetrahydrofuran (160 mL) and ammonium acetate (390 mg, 5.07 mmol). Sodium trisacetoxyborohydride (4.99 g, 23.6 mmol) was added in portions and the resulting suspension was stirred for 24 h. The solution was neutralized with an aqueous solution of sodium bicarbonate and extracted with ethyl acetate (3 × 75 mL). The combined organic layers were dried over magnesium sulfate and filtered, and the solvents were evaporated. The residue was purified by flash chromatography on silica (dichloromethane/methanol, 50:1 to 5:1) to give a mixture of siloxane dimers/oligomers and ligand 14b as a colorless powder.

A 100 mL, one-necked flask was charged in air with this crude material and tetrahydrofuran (80 mL). An aqueous solution of sodium hydroxide (2 M, 80 mL) was added and the mixture was stirred overnight. The layers were separated and the aqueous phase was extracted with ethyl acetate (3 × 40 mL). The combined organic layers were washed with brine (1 × 50 mL), dried over magnesium sulfate and filtered, and the solvents were evaporated to give the title compound as a white powder (3.65 g, 65%). HRMS (ESI) calc. for C₆₀H₅₆NO₃Si₃ [M - H]⁻: 922.35735; found: 922.35784. 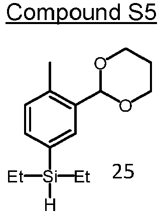 A 50 mL Schlenk flask was charged with compound S4 (4.00 g, 15.6 mmol) and tetrahydrofuran (30 mL). The solution was cooled to 0 °C and a solution of isopropylmagesium chloride lithium chloride complex (1.25 M in tetrahydrofuran, 4.78 mL, 6.22 mmol) was added dropwise. After stirring for 5 min, a solution of n-butyllithium (1.6 M in hexane, 7.78 mL, 12.4 mmol) was added dropwise and the resulting grey suspension was stirred for 1 h at 0 °C. A 250 mL, two necked round bottom flask equipped with a pressure equilibrating dropping funnel and connected to a vacuum/argon manifold was charged with Et₂SiH₂ (6.01 mL, 46.7 mmol) and tetrahydrofuran (20 mL). The resulting solution was cooled to 0 °C. The Grignard reagent was transferred into the dropping funnel with a LDPE tube and argon overpressure and then slowly added to the cooled diethylsilane solution. Once the addition was complete, the mixture was allowed to warm to ambient temperature and stirring was continued overnight. The reaction was carefully quenched with an aqueous, saturated solution of ammonium chloride (40 mL). The layers were separated and the aqueous phase was extracted with ethyl acetate (3 × 50 mL). The combined organic layers were dried over magnesium sulfate and filtered, and the solvents were evaporated. The residue was purified by flash chromatography on silica (hexane/ethyl acetate, 12:1) to give the title compound as a colorless oil (3.88 g, 94%). HRMS (EI) calc. for C₁₅H₂₄O₂Si [M]⁺ : 264.15401; found: 264.15419. 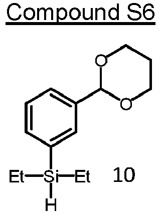 Prepared analogously from S3 (4.00 g, 16.5 mmol) as a colorless liquid (2.89 g, 70%).
HRMS (ESI) calc. for C₁₄H₂₂O₂SiNa [M + Na]⁺: 273.12823; found: 273.12808. 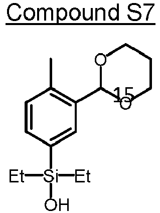 A 50 mL, one-necked round bottom flask was charged in air with compound S5 (3.87 g, 14.7 mmol) and dichloromethane (30 mL). The solution was cooled to 0 °C before m-chloroperoxybenzoic acid (77% *w*/*w*, 3.62 g, 16.1 mmol) was added in portions. After stirring at ambient temperature for 5 h, the mixture was transferred into a separation funnel and was washed with a saturated aqueous solution of sodium bicarbonate (2 × 60 mL) and aqueous sodium hydroxide (1 M, 60 mL). The organic layer was dried over magnesium sulfate and filtered, and the solvents were evaporated to give the title compound as a colorless liquid (3.84 g, 92%). HRMS (EI) calc. for C₁₅H₂₄O₃Si [M]⁺ : 280.14892; found: 280.14896. 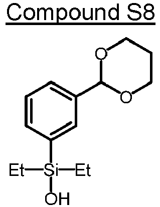 Prepared analogously from compound S6 (2.89 g, 11.5 mmol) as a colorless liquid (2.84 g, 93%).
HRMS (EI) calc. for C₁₄H₂₂O₃Si [M]⁺: 266.13327; found: 266.13357. 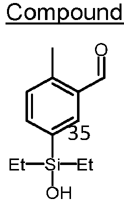 A 250 mL, one-neck round bottom flask equipped with a 50 mL dropping funnel was charged in air with compound S7 (3.87 g, 13.8 mmol) and tetrahydrofuran (120 mL). The solution was cooled to 0 °C before hydrochloric acid (12 M in water, 17.0 mL, 207 mmol) was added *via* the dropping funnel over 10 min. After 20 min at 0 °C, the solution was carefully neutralized with a saturated aqueous solution of sodium bicarbonate and the aqueous phase was extracted with dichloromethane (3 × 100 mL). The combined organic layers were dried over sodium sulfate and filtered, and the solvents were evaporated to give the title compound as a colorless liquid (2.72 g, 89%). Compound S9 is prone to siloxane formation and was immediately used in the subsequent step. HRMS (ESI) calc. for C₁₂H₁₈O₂SiNa [M + Na]⁺: 245.09683; found: 245.09693. 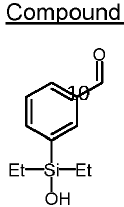 Prepared analogously from compound S8 (500 mg, 1.88 mmol) as a colorless liquid (3.22 g, 82%). This compound is prone to siloxane formation and was immediately used in the next step of the ligand synthesis. HRMS (EI) calc. for C₁₁H₁₆O₂Si [M]⁺ : 208.09141; found: 208.09143.

### Ligand S11

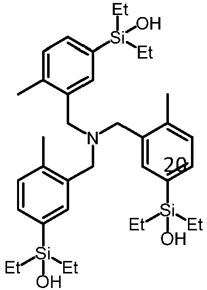

A 250 mL, two-necked round bottom flask connected to a vacuum/argon manifold was charged with compound S9 (2.72 g, 12.2 mmol), tetrahydrofuran (100 mL) and ammonium acetate (304 mg, 3.95 mmol). Sodium trisacetoxyborohydride (3.81 g, 18.0 mmol) was added in portions and the resulting suspension was stirred for 24 h. Additional sodium triacetoxyborohydride (1.00 g, 4.72 mmol) was then added and stirring continued for an additional 48 h. For work up, the solution was cooled to 0 °C and carefully neutralized with an aqueous solution of sodium bicarbonate. The mixture was concentrated *via* rotary evaporation until 50 mL remained and was then extracted with tert-butyl methyl ether (3 × 50 mL). The combined organic layers were washed with an aqueous solution of sodium hydroxide (1 M, 40 mL), dried over magnesium sulfate and filtered, and the solvents were evaporated. The residue was purified by flash chromatography on silica (hexane/ethyl acetate, 5:1 to 4:1) to give the title compound as a colorless powder (873 mg, 35%). HRMS (ESI) calc. for C₃₆H₅₆NO₃Si₃ [M - H]⁻: 634.35736; found: 634.35773.

### Ligand S12

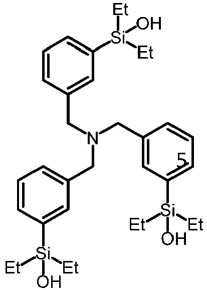

Prepared analogously from compound S10 (3.47 g, 15.3 mmol) as a colorless oil (960 mg, 33%).
HRMS (ESI) calc. for C₃₃H₅₂NO₃Si₃ [M + H]⁺ : 594.32496; found: 594.32459.

### Complexes

### Complex 15b

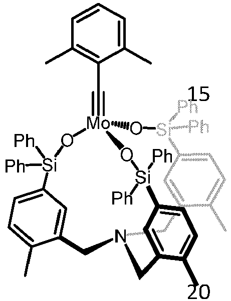

A 250 mL Schlenk flask was charged with ligand 14b (855 mg, 0.93 mmol), which was azeotropically dried with benzene (2 × 4 mL) to remove any residual water. The compound was suspended in toluene (90 mL) and a solution of the alkylidyne complex 9 (400 mg, 0.93 mmol) in toluene (10 mL) was added dropwise. After stirring for 1.5 h, all volatile materials were removed *in vacuo* to give the title complex as a yellow powder (1.04 g, 98%). Orange crystals suitable for single-crystal X-ray diffraction were grown from a concentrated solution in benzene. HRMS (ESI) calc. for C₆₉H₆₃MoNO₃Si₃Na [M + Na]⁺: 1158.30620; found: 1158.30562.

### Complex 15a

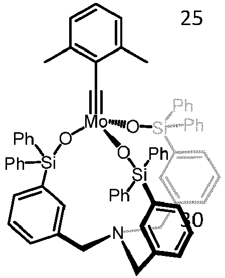

A 50 mL Schlenk flask was charged with ligand 14a (204 mg, 0.23 mmol), which was azeotropically dried with benzene (3 × 1.5 mL) to remove residual water. The compound was suspended in toluene (30 mL) and a solution of the alkylidyne complex 9 (100 mg, 0.93 mmol) in toluene (8 mL) was added dropwise. After stirring for 3.5 h, all volatile materials were removed *in vacuo* to give the title complex as a yellow powder (238 mg, 94%). HRMS (ESI) calc. for C₆₆H₅₈MoNO₃Si₃ [M + H]⁺: 1094.27731; found: 1094.27686.

### Complex 17a

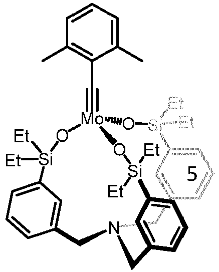

A 100 mL Schlenk flask was charged with ligand S12 (339 mg, 0.52 mmol, 90% purity), which was azeotropically dried with benzene (2 × 2 mL) to remove residual water. The compound was dissolved in toluene (50 mL) and a solution of the akylidyne complex 9 (222 mg, 0.51 mmol) in toluene (6 mL) was added dropwise. After stirring for 1 h, all volatile materials were removed *in vacuo* to give a brown residue. This residue was dissolved in dichloromethane (3 mL) and an excess of pentane (50 mL) was added. The title complex precipitated from the solution after 3 d at -78 °C as a yellow powder and was collected by filtration (336 mg, 81%). HRMS (ESI) calc. for C₄₂H₅₈MoNO₃Si₃ [M + H]⁺: 806.27731; found: 806.27657.

### Complex 17b

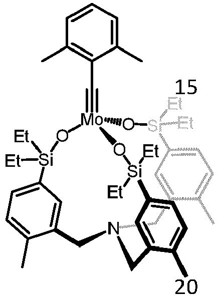

A 100 mL Schlenk flask was charged with ligand S11 (300 mg, 0.47 mmol), which was azeotropically dried with benzene (2 × 2 mL) to remove residual water. The compound was suspended in toluene (45 mL) and a solution of the alkylidyne complex 9 (204 mg, 0.47 mmol) in toluene (8 mL) was added dropwise. After stirring for 3.5 h, all volatile materials were removed *in vacuo* to give a brown residue. The crude material was repeatedly triturated with pentane and then dried under high vacuum to give the toluene-free complex 17b as a yellow powder (399 mg, 99%). HRMS (ESI) calc. for C₄₆H₆₄MoNO₃Si₃ [M + H]⁺: 848.32426; found: 848.32437.

### Adducts

### Adduct 16a

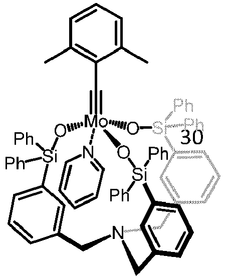

A 25 mL Schlenk flask was charged with complex 15a (835 mg, 0.76 mol) and dichloromethane (15 mL). Pyridine (80 µL, 0.99 mmol) was added to the orange solution, causing an immediate color change to dark purple. After stirring for 1 h, all volatile materials were removed *in vacuo* to give a purple powder, which was washed with pentane (2 × 6 mL) and dried in vacuum to yield the title adduct (787 mg, 88%). Purple crystals suitable for single-crystal X-ray diffraction were grown from a dichloromethane/pentane solution by slow evaporation of the solvents.

### Adduct 16b

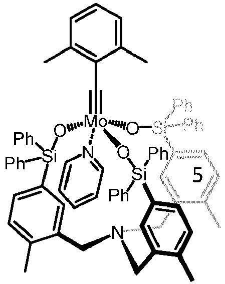

A 10 mL Schlenk flask was charged with complex 15b (98 mg, 87 µmol) and dichloromethane (2 mL). Pyridine (11 µL, 0.13 mmol) was added to the orange solution, causing an immediate color change to dark purple. After stirring for 2 h, all volatile materials were removed *in vacuo* to give a purple powder which was washed with pentane (3 × 1.5 mL) and dried in vacuum to yield adduct 16b (81 mg, 77%). Purple crystals suitable for single-crystal X-ray diffraction were grown from a dichloromethane/pentane solution by slow evaporation of the solvents. HRMS (ESI) calc. for C₇₄H₆₈MoN₂O₃Si₃ [M]⁺: 1214.35863; found: 1214.35929.

### 3-Bromopyridine Adduct 16c

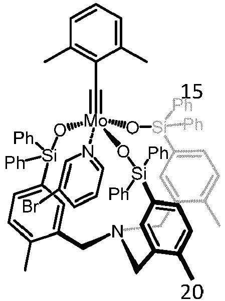

A 10 mL Schlenk flask was charged with complex 15b (49 mg, 43 µmol) and dichloromethane (1.5 mL). 3-Bromopyridine (7 µL, 73 µmol) was added to the orange solution, causing an immediate color change to dark purple. After 15 min, pentane (7 mL) was added and the solution was cooled to -78 °C. The supernatant solution was separated from the precipitate by transfer into a new Schlenk flask using a filter-capped LDPE tube and argon overpressure. The solvents were removed in a constant stream of argon. The resulting purple solid was washed with pentane (3 × 1.5 mL) at 0 °C and dried in a gentle stream of argon to give the title adduct (48 mg, 85%).

### 3,5-Dibromo-pyridine Adduct 16d

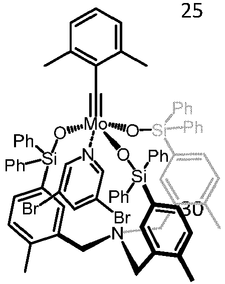

A 10 mL Schlenk flask was charged with complex 15b (75 mg, 66 µmol) and dichloromethane (1.5 mL). 3,5-Dibromopyridine (23 mg, 99 µmol) was added to the orange solution, causing an immediate color change to dark red. After stirring for 1 h, pentane (5 mL) was added. The supernatant solution was separated from the precipitate by transfer into a new Schlenk flask using a filter-capped LDPE tube and argon overpressure. The solvents were removed in a constant stream of argon. The resulting purple solid was washed with pentane (3 × 2 mL) at 0 °C and dried in a gentle stream of argon to give the title adduct (75 mg, 83%).

### 4-Pvrrolidinopvridine Adduct 16e

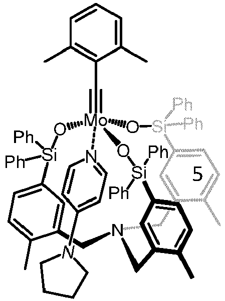

A 10 mL Schlenk flask was charged with complex 15b (76 mg, 67 µmol) and dichloromethane (1.5 mL). 4-Pyrrolidino-pyridine (15 mg, 0.1 mmol) was added to the orange solution, causing an immediate color change to dark purple. After stirring for 3 h, all volatile materials were removed *in* vacuo. The purple powder was washed with pentane (2 × 2 mL) to give the title adduct (71 mg, 55 µmol, 83%) containing traces of free 4-pyrrolidino-pyridine and hydrolyzed ligand. The purity of the sample was only ca. 93% (NMR).

### Alkyne Metathesis Reactions

### Representative Procedure for Alkyne Homo-Metathesis

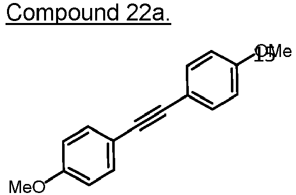

A 10 mL Schlenk flask was charged with 1-(4-methoxyphenyl)-1-propyne (21, 37 mg, 0.25 mmol), powdered molecular sieves (250 mg, 5Å) and toluene (1.25 mL). Adduct 16a (6 mg, 5 µmol, 2 mol%) was weight out in air and added in one portion to the mixture. After stirring for 12 h at ambient temperature, the reaction was quenched with ethanol (1 mL) and the mixture was filtered through a short pad of Celite^{®} which was carefully rinsed with ethyl acetate (20 mL) and dichloromethane (10 mL). The combined filtrates were evaporated and the residue was purified by flash chromatography on silica gel (hexane/ethyl acetate, 70:1 to 50:1) to give the title compound as a colorless solid (27 mg, 91%).

The spectroscopic data was in agreement with the literature.

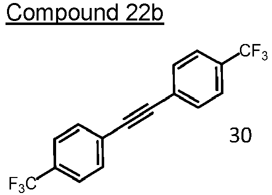

Prepared analogously with adduct 16a as the catalyst (6 mg, 5 µmol, 2 mol%) at 50 °C. Purified by flash chromatography (hexane) to give the title compound as a colorless solid (35 mg, 89%).

The spectroscopic data was in agreement with the literature.

### Compound 22c.

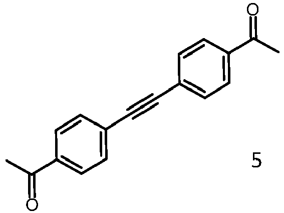

Prepared analogously with adduct 16a as the catalyst (6 mg, 5 µmol, 2 mol%) at 50 °C. Purified by flash chromatography (hexane/ethyl acetate, 8:2 to 1:1) to give the title compound as a colorless solid (28 mg, 85%).

The spectroscopic data was in agreement with the literature.

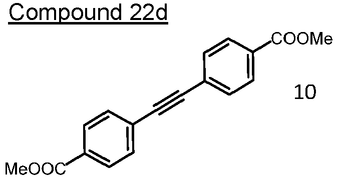

Prepared analogously with adduct 16a as the catalyst (6 mg, 5 µmol, 2 mol%) at 50 °C. Purified by flash chromatography (hexane/dichloromethane, 1:1) to give the title compound as a colorless solid (36 mg, 98%)

The spectroscopic data was in agreement with the literature.

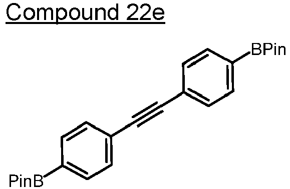

Prepared analogously with adduct 16a as the catalyst (6 mg, 5 µmol, 2 mol%) at 50 °C. Purified by flash chromatography (hexane/dichloromethane, 10:1 to 8:2) to give the title compound as a colorless solid (38 mg, 71%).
HRMS (ESI) calc. for C₂₆H₃₂B₂O₄Na [M + Na]⁺: 453.23794; found: 453.23815. The spectroscopic data was in agreement with the literature.

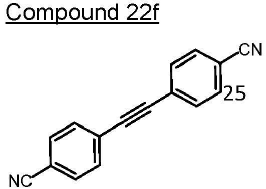

Prepared analogously with adduct 16a as the catalyst (15 mg, 13 µmol, 5 mol%) at 50 °C. Purified by flash chromatography (hexane/ethyl acetate 12:1 to 3:2 to 1:1) to give crude product containing the co-polar ligand. To remove the impurity, the crude product was dissolved in ethyl acetate (30 mL) and the organic layer washed with aqueous hydrogen chloride (3 × 10 mL, 3 M). The organic layer was dried over magnesium sulfate and filtered, and the solvents were evaporated to give the title compound as a colorless solid (26 mg, 91 %). The spectroscopic data was in agreement with the literature.

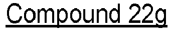

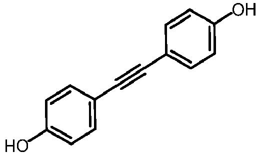

Prepared analogously with adduct 16a as the catalyst (15 mg, 13 µmol, 5 mol%) at 50 °C. Purified by flash chromatography (hexane/ethyl acetate, 8:2 to 1:1) to give the title compound as a red solid (36 mg, 98%). The spectroscopic data was in agreement with the literature.

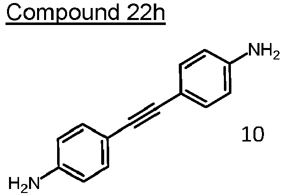

Prepared analogously with complex 17 as the catalyst (10 mg, 13 µmol, 5 mol%) at 110 °C. Purified by flash chromatography (hexane/ethyl acetate, 3:1 to 1:1) to give the title compound as an orange solid (24 mg, 71%). The spectroscopic data was in agreement with the literature.

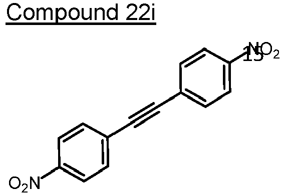

Prepared analogously with complex 17 as the catalyst (15 mg, 17 µmol, 7 mol%) at 110 °C. Purified by flash chromatography (hexane/ethyl acetate, 4:1) to give the title compound as a yellow solid (15 mg, 45%). HRMS (EI) calc. for C₁₄H₈N₂O₄ [M]⁺: 268.04786; found: 258.04825. The spectroscopic data was in agreement with the literature.

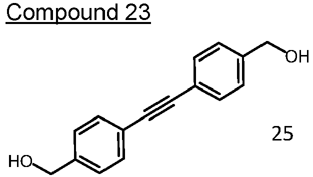

Prepared analogously with adduct 16a as the catalyst (15 mg, 13 µmol, 5 mol%) at 50 °C. Purified by flash chromatography (dichloromethane/methanol 98:2) to give the title compound as a colorless solid (24 mg, 81%).
The spectroscopic data was in agreement with the literature.

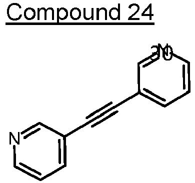

Prepared analogously with adduct 16a as the catalyst (6 mg, 5 µmol, 2 mol%) at 90 °C. Purified by flash chromatography (hexane/ethyl acetate, 3:2 to 1:1) to give the title compound as a yellowish solid (20 mg, 89%). The spectroscopic data was in agreement with the literature.

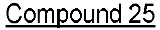

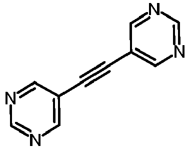

Prepared analogously with adduct 16a as the catalyst (6 mg, 5 µmol, 2 mol%) at 100 °C. Purified by flash chromatography (dichloromethane/methanol, 95:5) to give the title compound as a colorless solid (10 mg, 44%). The spectroscopic data was in agreement with the literature.

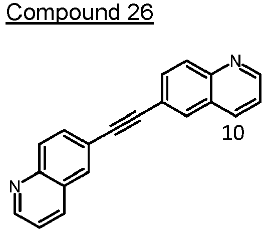

Prepared analogously with adduct 16a as the catalyst (6 mg, 5 µmol, 2 mol%) at 50 °C. Purified by flash chromatography (dichloromethane/methanol, 99:1 to 98:2) to give the title compound as a colorless solid (28 mg, 80%). HRMS (EI) calc. for C₂₀H₁₂N₂ [M]⁺: 280.09950; found: 280.09974. The spectroscopic data was in agreement with the literature.

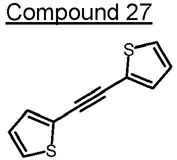

Prepared analogously with adduct 16a as the catalyst (6 mg, 5 µmol, 2 mol%) at 50 °C. Purified by flash chromatography (hexane/dichloromethane, 100:1) to give the title compound as a colorless solid (21 mg, 88%). The spectroscopic data was in agreement with the literature.

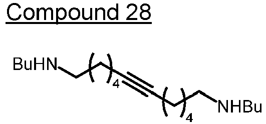

Prepared analogously with adduct 16a as the catalyst (15 mg, 13 µmol, 5 mol%) at 50 °C. Purified by flash chromatography (dichloromethane/methanol, 99:1 to 95:5 with 2% triethylamine) to give the title compound as a brown oil (31 mg, 80%). HRMS (ESI) calc. for C₂₀H₄₁N₂ [M + H]⁺ : 309.32643; found: 309.32658. The spectroscopic data was in agreement with the literature.

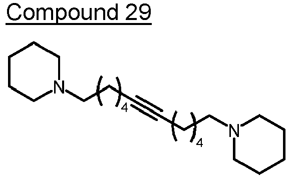

Prepared analogously with adduct 16a as the catalyst (6 mg, 5 µmol, 2 mol%) at 50 °C. Purified by flash chromatography (hexane/ethyl acetate, 3:1 with trimethylamine 5%) to give the title compound as a yellow oil (35 mg, 80%). The spectroscopic data was in agreement with the literature.

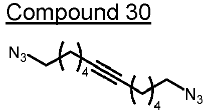

Prepared analogously with adduct 16a as the catalyst (6 mg, 5 µmol, 2 mol%) at 50 °C. Purified by flash chromatography (hexane/ethyl acetate, 50:1) to give the title compound as a clear oil (24 mg, 77%). The spectroscopic data was in agreement with the literature.

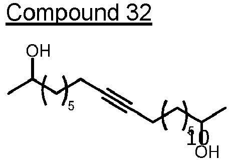

Prepared analogously with adduct 16a as the catalyst (15 mg, 13 µmol, 5 mol%) at 50 °C. Purified by flash chromatography (dichloromethane/methanol, 98:2 to 90:10) to give the title compound as a yellow oil (29 mg, 82%). The spectroscopic data was in agreement with the literature.

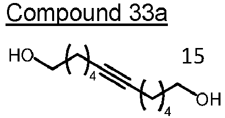

Prepared analogously with adduct 16a as the catalyst (15 mg, 13 µmol, 5 mol%) at 50 °C using 4 Å molecular sieves (250 mg) instead of 5 Å molecular sieves. Purified by flash chromatography (hexane/ethyl acetate, 1:1) to give the title compound as a colorless solid (16 mg, 65%). HRMS (ESI) calc. for C₁₂H₂₃O₂[M + H]⁺: 199.16962; found: 199.16956. The spectroscopic data was found to be in agreement with the data reported in the literature.

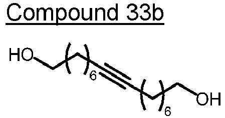

Prepared analogously with adduct 16a as the catalyst (15 mg, 13 µmol, 5 mol%) at 50 °C. Purified by flash chromatography (hexane/ethyl acetate, 2:3) to give the title compound as a colorless solid (25 mg, 79%). HRMS (ESI) calc. for C₁₆H₃₀O₂Na [M + Na]⁺: 277.21387; found: 277.21381. The spectroscopic data was in agreement with the literature.

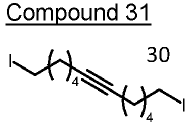

A 10 mL Schlenk flask was charged with 8-iodooct-2-yne (59 mg, 0.25 mmol), powdered molecular sieves (250 mg, 5Å) and toluene (1.15 mL). Adduct 16a (6 mg, 5 µmol, 2 mol%) was weight out in air and added in one portion. A stock solution of triphenylborane (0.01 M in toluene, 0.5 mL, 5 µmol, 2 mol%) was added and the mixture was stirred for 1 h at ambient temperature. The reaction was quenched with ethanol (1 mL) and the mixture was filtered through a short pad of Celite^{®} which was carefully rinsed with ethyl acetate (20 mL) and dichloromethane (10 mL).

The solvents were evaporated and the residue was purified by flash chromatography on silica (hexane/ethyl acetate, 95:5) to give the title compound as a yellow oil (48 mg, 82%). The spectroscopic data was in agreement with the literature.

### Representative Procedure for Ring Closing Alkyne Metathesis

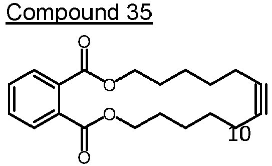

A 100 mL Schlenk flask was charged with di(oct-6-yn-1-yl) phthalate (38 mg, 0.10 mmol), powdered molecular sieves (250 mg, 5Å) and toluene (50 mL). Adduct 16a (6 mg, 5 µmol, 5 mol%) was weight out in air and added in one portion, and the mixture was stirred for 12 h at ambient temperature. The reaction was quenched with ethanol (5 mL) and the mixture was filtered through a short pad of Celite^{®} which was carefully rinsed with ethyl acetate (20 mL) and dichloromethane (10 mL). The solvents were evaporated and the residue was purified by flash chromatography on silica gel (hexane/ethyl acetate, 10:1) to give the title compound as a yellow oil (30 mg, 91%). The spectroscopic data was in agreement with the literature.

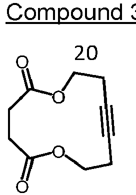

Prepared analogously with adduct 16a as the catalyst (2 mg, 2 µmol, 2 mol%) at 50 °C. Purified by flash chromatography (hexane/ethyl acetate, 9:1) to give the title compound as a colorless oil (19 mg, 97%).. The spectroscopic data was in agreement with the literature.

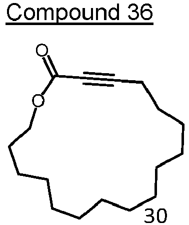

Prepared analogously with adduct 16a as the catalyst (8.5 mg, 7 µmol, 10 mol%) at reflux over 2 h. Purified by flash chromatography (hexane/ethyl acetate, 30:1) to give the title compound as a colorless solid (26 mg, 98%). The spectroscopic data was in agreement with the literature.

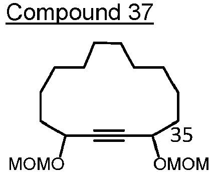

Prepared analogously with complex 17b as the catalyst (17 mg, 20 µmol, 20 mol%) at reflux over 4 h. The complex was dissolved in a separate Schlenk flask in toluene (1 mL) and the solution added dropwise to the solution of the diyne at 110 °C. The crude product was purified by flash chromatography (hexane/ethyl acetate, 10:1 to 7:3) to give the title compound as a yellow oil (24 mg, 77%). HRMS (ESI) calc. for C₁₈H₃₂O₄Na [M + Na]⁺ : 335.21928; found: 335.21918. The spectroscopic data was in agreement with the literature.

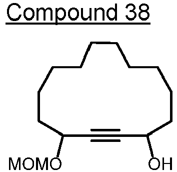

Prepared analogously with complex 16a as the catalyst (23 mg, 20 µmol, 20 mol%) at reflux over 4 h. The catalyst was dissolved in a separate Schlenk flask in toluene (1 mL) and the solution added dropwise to the solution of the diyne at 110 °C. The crude product was washed with an aqueous HCl (3 M, 3 × 10 mL), the organic phase was dried over magnesium sulfate and filtered, and the solvents were evaporated. Purification of the residue by flash chromatography (toluene/tert-butyl methyl ether, 8:2) gave the title compound as a colorless solid (16 mg, 60%). HRMS (ESI) calc. for C₁₆H₂₈NaO₃ [M + Na]⁺ : 291.19307; found: 291.19290. The spectroscopic data was in agreement with the literature.

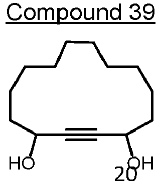

Prepared analogously with complex 16a as the catalyst (23 mg, 20 µmol, 20 mol%) at reflux temperature. The complex was dissolved in a separate Schlenk flask in toluene (1 mL) and the solution was added dropwise to the solution of the diyne at 110 °C. The crude product was purified by flash chromatography (hexane/ethyl acetate 9:1) to give the title compound as a colorless solid (10 mg, 46%). HRMS (CI) calc. for C₁₄H₂₈NO₂ [M + NH₄]⁺: 242.21145; found: 242.21154. The spectroscopic data was in agreement with the literature.

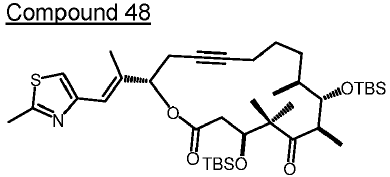

A 50 mL Schlenk flask was charged with diyne 47 (10 mg, 13 µmol), powdered molecular sieves (250 mg, 5Å) and toluene (7 mL). Adduct 16a (1 mg, 0.5 µmol, 5 mol%) was weight out in air and dissolved in toluene (1 mL). The catalyst solution was added dropwise to the solution of the substrate at 60 °C and stirring was continued for 2 h at this temperature. The reaction was quenched with ethanol (5 mL) before the mixture was cooled to ambient temperature and filtered through a short pad of Celite^{®} which was carefully rinsed with ethyl acetate (20 mL) and dichloromethane (10 mL). The solvents were evaporated and the residue was purified by flash chromatography on silica (pentane/diethyl ether, 10:1 to 7:3) to give the title compound as a colorless solid (8 mg, 81%). HRMS (ESI) calc. for C₃₈H₆₅NO₅Si₂SNa [M + Na]⁺: 726.40142; found: 726.40156. The spectroscopic data was in agreement with the literature.

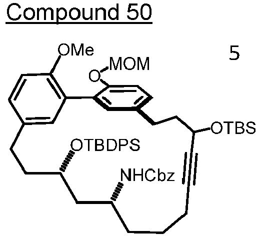

A 50 mL Schlenk flask was charged with diyne 49 (10 mg, 10 µmol), powdered molecular sieves (250 mg, 5Å) and toluene (5 mL). The mixture was heated to 50 °C. Adduct 16a (0.5 mg, 0.5 µmol, 5 mol%) was weight out in air and added in one portion. After stirring for 1.5 h at 50 °C, the reaction was quenched with ethanol (5 mL) before the mixture was cooled to ambient temperature and filtered through a short pad of Celite^{®} which was carefully rinsed with ethyl acetate (20 mL) and dichloromethane (10 mL). The solvents were evaporated and the residue was purified by flash chromatography on silica (hexane/ethyl acetate, 6:1) to give the title compound as a colorless oil (6 mg, 65%). The two diastereomers of compound 50 exists in solution as two rotamers each. HRMS (ESI) calc. for C₅₈H₇₅O₇NSi₂Na [M + Na]⁺ : 976.49743; found: 976.49749. The spectroscopic data was in agreement with the literature.

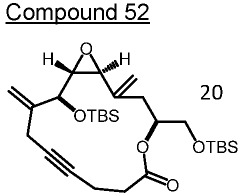

A 50 mL Schlenk flask was charged with diyne 51 (10 mg, 17 µmol), powdered molecular sieves (250 mg, 5Å) and toluene (9 mL). Adduct 16a (2 mg, 2 µmol, 10 mol%) was weight out in air and dissolved in toluene (1 mL). The catalyst solution was added dropwise to the solution of the substrate at 60 °C and stirring was continued for 1.5 h at this temperature. The reaction was quenched with ethanol (5 mL) before the mixture was cooled to ambient temperature and filtered through a short pad of Celite^{®} which was carefully rinsed with ethyl acetate (20 mL) and dichloromethane (10 mL). The solvents were evaporated and the residue was purified by flash chromatography on silica (hexane/ethyl acetate, 22:1) to give the title compound as a colorless solid (9 mg, 94%). HRMS (ESI) calc. for C₂₈H₄₈O₅Si₂Na [M + Na]⁺: 543.29325; found: 543.29340. The spectroscopic data was in agreement with the literature.

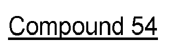

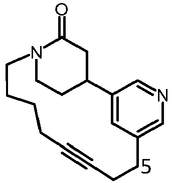

A 50 mL Schlenk flask was charged with diyne 53 (20 mg, 59 µmol), powdered molecular sieves (500 mg, 5Å) and toluene (30 mL). Adduct 16a (21 mg, 18 µmol, 30 mol%) was weight out in air and dissolved in toluene (1 mL) in a separate Schlenk flask. The catalyst solution was added dropwise to the solution of the substrate stirred at reflux temperature. After 1.5 h, the reaction was quenched by the addition of ethanol (5 mL), the mixture was cooled to ambient temperature and filtered through a short pad of Celite^{®} which was carefully rinsed with ethyl acetate (20 mL) and dichloromethane (10 mL). The solvents were evaporated and the residue was purified by flash chromatography on silica gel (dichloromethane/methanol, 95:5) to give the title compound as a colorless solid (12 mg, 71%). HRMS (EI) calc. for C₁₈H₂₂N₂O [M]⁺: 282.17266; found: 282.17293. The spectroscopic data was in agreement with the literature.

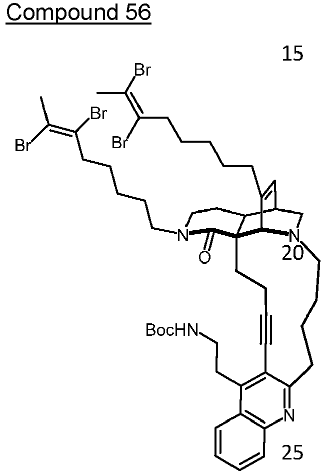

A 25 mL Schlenk flask was charged with diyne 55 (10 mg, 8.7 µmol), powdered molecular sieves (400 mg, 5Å) and toluene (5 mL). Adduct 16a (3 mg, 2.6 µmol, 30 mol%) was weight out in air and dissolved in toluene (1 mL). The catalyst solution was added dropwise to the solution of the substrate stirred at reflux temperature. After stirring for 2 h, the reaction was quenched with ethanol (5 mL) before the mixture was cooled to ambient temperature and filtered through a short pad of Celite^{®} which was carefully rinsed with ethyl acetate (20 mL) and dichloromethane (10 mL). The solvents were evaporated and the residue was purified by flash chromatography on silica (hexane/ethyl acetate, 4:1 with trimethylamine 5%) to give the title compound as a pale brown oil (8 mg, 81%). HRMS (ESI) calc. for C₅₀H₆₇O₃N₄Br₄ [M + H]⁺: 1087.19412; found: 1087.19569. The spectroscopic data was in agreement with the literature.

### Conclusions

A new generation of molybdenum alkylidynes is described in the present invention, which serve as catalysts for alkyne metathesis reactions of all sorts. In particular, the derived pyridine adducts of type 16 are easy to make on scale, can be routinely weighed and handled in air, and can be stored for extended periods of time outside a glovebox in a desiccator or in a screw-capped vial in a freezer. When dissolved in toluene, spontaneous dissociation of the stabilizing pyridine ligand releases the active species: its performance is excellent and the functional group tolerance is outstanding. Indeed, the new catalysts meet high standards in terms of reactivity and selectivity and rival the best alkyne metathesis catalysts known to date. Their functional group tolerance is remarkable, in that they embrace a host of polar and apolar groups, different protic sites, as well as numerous basic functionalities.

## Claims

1. Metal-organic compound of the general Formula (I), 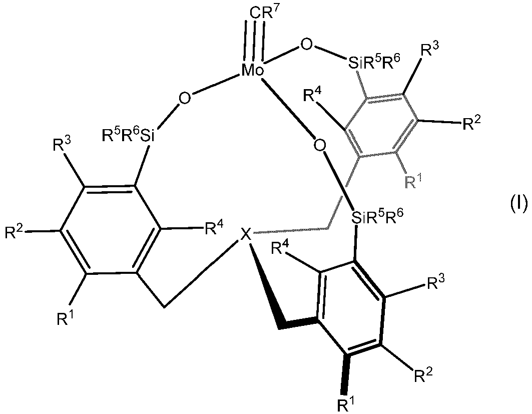 Wherein:
X represents N, CR^{x}, SiR^{x}, wherein R^{x} is selected from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more heteroatoms;
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl, or
(R1 and R2) or (R2 and R3) on the same phenyl ring form an aromatic or heteroaromatic ring structure conjugated with the phenyl ring and having a total of 10 to 18 carbon atoms in the ring structure wherein one to three ring carbon atoms may be replaced by nitrogen, which aromatic or heteroaromatic ring structure may be substituted by one or more heteroatoms,
R4 is selected from H, methyl or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, which alkyl, cycloalkyl, aromatic or heteroaromatic ring structure may be substituted by one or more substituents selected from C1 to C6 alkyl, 6-14 membered aryl or heteroatoms,
R7 is selected from C1-C12-alkyl, C3-C12-cycloalkyl, 6-18 membered aryl, or 5-10 membered heteroaryl which alkyl, cycloalkyl, aromatic or heteroaromatic ring structure may be substituted by one or more substituents selected from C1 to C6 alkyl, 6-14 membered aryl or heteroatoms.

2. Metal-organic compound of Formula (I) according to claim 1, 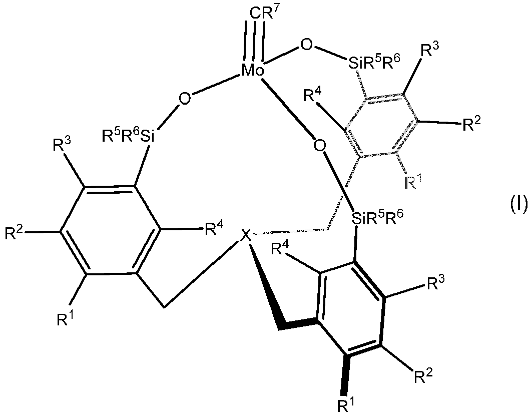 Wherein:
X represents N, CR^{x}, SiR^{x}., wherein R^{x} is selected from C1-C12-alkyl, C3-C12-cycloalkyl, 6-18 membered aryl, or 5-10 membered heteroaryl,
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl,
R4 is selected from H, methyl, or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, 6-18 membered aryl, or 5-10 membered heteroaryl, which alkyl, cycloalkyl, aromatic or heteroaromatic ring structure may be substituted by one or more substituents selected from C1 to C6 alkyl, 6-14 membered aryl or heteroatoms
R7 is selected from C1-C12-alkyl, C3-C12-cycloalkyl or 6-18 membered aryl which alkyl, cycloalkyl, or aromatic ring structure may be substituted by one or more substituents selected from C1 to C6 alkyl, 6-14 membered aryl or heteroatoms.

3. Metal-organic compound of Formula (I) according to claim 1, 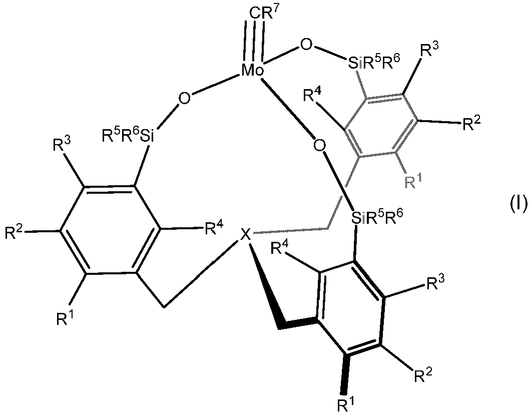 Wherein
X is N,
R1, R2 and R3 may be equal or different from one another and are selected independently from H, C1-C12-alkyl, or C3-C12-cycloalkyl,
R4 is selected from H, methyl or ethyl,
R5 and R6 may be equal or different from one another and are selected independently from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more substituents selected from C1 to C6 alkyl, 6-14 membered aryl or heteroatoms
R7 is selected from C1-C12-alkyl or 6-18 membered aryl, which alkyl or aromatic ring structure may be substituted by one or more substituents selected from C1 to C6 alkyl, 6-14 membered aryl or heteroatoms.

4. Metal-organic compound of Formula (I) according to claim 1, 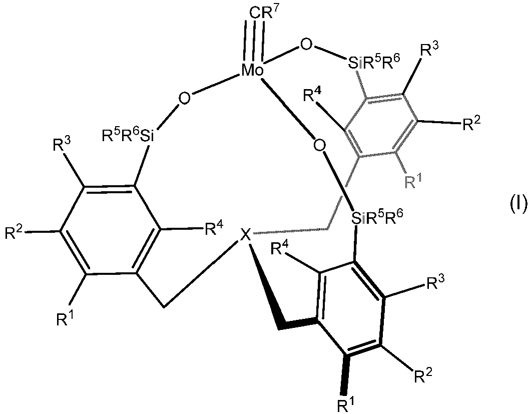 Wherein
Xis N,
R1, R2 and R3 are selected independently from H or C1-C6-alkyl,
R4 is H,
R5 and R6 may be equal or may be different from one another and represent C1-C12-alkyl or 6-18 membered aryl,
R7 represents C1-C12-alkyl or 6-18 membered aryl, which alkyl or aromatic ring structure may be substituted by one or more substituents selected from C1 to C6 alkyl, 6-14 membered aryl or heteroatoms.

5. Adduct of a metal-organic compound of Formula (I) as claimed in any one of claims 1 to 4, with one adduct- forming compound, selected from.
a nitrogen-containing 5 to 10 membered heteroaryl compound, said heteroaryl optionally being substituted by one or more C1- C6-alkyl, halogen, -CN, -CF₃, -N(C₁-C₆ alkyl)₂ or -NO₂ substituents, or
PR^{P}₃ wherein R^{P} is independently selected from C1-C6-alkyl or 6-18 membered aryl, said alkyl or aryl optionally being substituted by one or more C1- C3-alkyl, halogen, - CN, -CF₃, -N(C₁-C₃ alkyl)₂ or -NO₂ substituents.

6. Adduct of a metal-organic compound of Formula (I) according to claim 5, wherein the adduct- forming compound is pyridine, optionally being substituted by one or more C1 -C6-alkyl, halogen, -CN, -CF₃, -N(C₁-C₆ alkyl)₂ or -NO₂ substituents with the proviso that at least one of the pyridine substituents on the 2 and -6 positions is H, the adduct being represented by Formula (II): 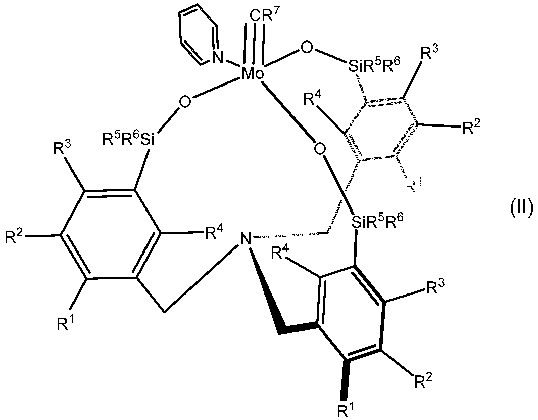 wherein R1 to R7 have the meaning as defined in any one of claims 1 to 4.

7. Process for preparing a metal-organic compound of the general Formula (I), 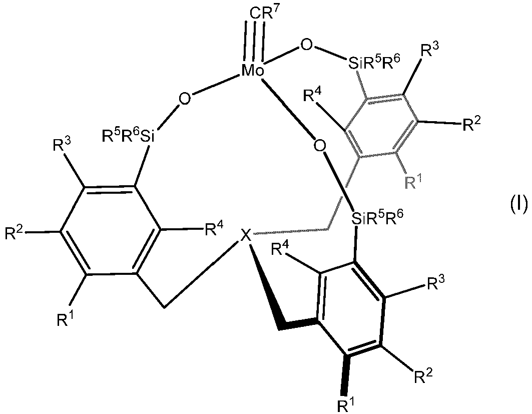 Wherein:
X represents N, CR^{x}, SiR^{x}, wherein R^{x} is selected from C1-C12-alkyl, C3-C12-cycloalkyl, 6-18 membered aryl, or 5-10 membered heteroaryl, which alkyl, cycloalkyl, aromatic or heteroaromatic ring structure may be substituted by one or more heteroatoms;
R1, R2 and R3 may be equal or may be different from one another and are selected independently from H, C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, halogen, nitro, cyano, trifluoromethyl, or -COOR⁸, wherein R⁸ is selected from C1-C12-alkyl, C3-C12-cycloalkyl, 6-18 membered aryl, or 5-10 membered heteroaryl, or
(R1 and R2) or (R2 and R3) on the same phenyl ring may form an aromatic or heteroaromatic ring structure conjugated with the phenyl ring and having a total of 10 to 18 carbon atoms in the ring structure wherein one to three ring carbon atoms may be replaced by nitrogen, which aromatic or heteroaromatic ring structure may be substituted by one or more heteroatoms,
R4 is selected from H, methyl or ethyl,
R5 and R6 may be equal or different from one another and are selected independently from C1-C12-alkyl, C3-C12-cycloalkyl, 6-18 membered aryl, 5-10 membered heteroaryl, C1-C12-alkyloxy, C3-C12-cycloalkyloxy, C6-C20-aryloxy, di-C1-C4-alkylamino, which alkyl, cycloalkyl, aromatic or heteroaromatic ring structure may be substituted by one or more heteroatoms,
R7 is selected from C1-C12-alkyl, C3-C12-cycloalkyl, 6-18 membered aryl, or 5-10 membered heteroaryl which alkyl, cycloalkyl, aromatic or heteroaromatic ring structure may be substituted by one or more substituents selected from C1 to C6 alkyl, 6-14 membered aryl or heteroatoms;
comprising the steps of reacting a Molybdenum complex of the Formula (III) with a compound of the Formula (IV) to yield a compound of Formula (I). 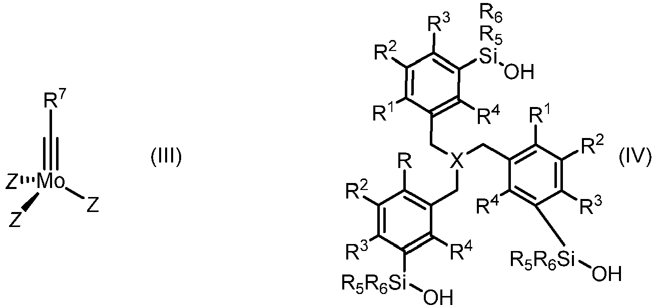 wherein Z represents C1-C6 alkyl, -O-C1-C6 alkyl, -N(C1-C6 alkyl)₂, -N(6-18 membered aryl)₂, or-N[(C1-C6 alkyl)(6-18 membered aryl)] and wherein X and R1 to R7 have the meaning as defined in any one of claims 1 to 4.

8. Use of a metal-organic compound of Formula (I) as defined in any of claims 1 to 4 as a catalyst for alkyne metathesis reactions.

9. Use of an adduct of a metal-organic compound of Formula (I) as defined in any of claims 5 to 6 as a catalyst for alkyne metathesis reactions.
